(19) Europäisches Patentamt
European Patent Office
Office européen des brevets

(11) EP 4 549 570 A1

(12) EUROPEAN PATENT APPLICATION
published in accordance with Art. 153(4) EPC

(43) Date of publication:
**07.05.2025 Bulletin 2025/19**

(21) Application number: **22948513.1**

(22) Date of filing: **30.06.2022**

(51) International Patent Classification (IPC):
***C12N 15/53*** (2006.01) ***C12N 9/02*** (2006.01)
***C12N 15/62*** (2006.01) ***C07K 14/02*** (2006.01)

(52) Cooperative Patent Classification (CPC):
**C07K 14/02; C12N 9/0004; C12N 15/62**

(86) International application number:
**PCT/CN2022/102827**

(87) International publication number:
**WO 2024/000408 (04.01.2024 Gazette 2024/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **DGI Tech (Qing Dao) Co., Limited
Qingdao, Shandong 266555 (CN)**

(72) Inventors:
- **PAN, Lulu
Qingdao, Shandong 266555 (CN)**
- **NI, Ming
Qingdao, Shandong 266555 (CN)**

(74) Representative: **Kransell & Wennborg KB
P.O. Box 27834
115 93 Stockholm (SE)**

Remarks:
The complete document including Reference Table(s) and the Sequence Listing(s) can be downloaded from the EPO website

# (54) LUCIFERASE MUTANT AND USE THEREOF

(57) Provided are a luciferase mutant and the use thereof, and specifically provided are a copepod luciferase mutant and the use thereof. By means of performing protein directed evolution on Pxluc, a mutant which has more than 2-fold increased substrate specificity to ZS26/F-CTZ and a mutant which has more than 4-fold increased substrate specificity to ZS2/F-CTZ are obtained. The luciferase can be subjected to prokaryotic expression, the purification process is simple, and large-scale production is facilitated. The luciferase has similar luminescence to Gluc, is easy to detect, and has broad application prospects in the fields of basic scientific research, biological monitoring, biochemical diagnosis, etc.

EP 4 549 570 A1

## Description

### FIELD

The present disclosure relates to the field of biotechnology. In particular, the present disclosure relates to luciferase mutant and use thereof, particularly copepod luciferase mutant and use thereof.

### BACKGROUND

Luciferases are a class of enzymes capable of catalyzing the oxidative luminescence of luciferin or aliphatic aldehydes and widely exist in insects, bacteria, fungi, and marine organisms. Luciferases have become important tools in scientific research, and have been widely used in the fields of life science research, genome sequencing and analysis techniques, clinical medicine and forensic detection, drug screening, environmental monitoring, and enzyme-linked detection. Due to the characteristics of self-luminescence, luciferase is often used in the fields of living-cell detection, protein-protein interaction, protein localization, small interfering RNA silencing, and high-throughput drug screening. In the field of biological monitoring technology, luciferase can be used to detect the presence or absence of chemical contaminants, and it has broad application prospects in immunodetection and biochemical diagnosis. In addition, as a reporter gene to detect the expression intensity and transcriptional regulation of exogenous genes under different promoters, a combination of multiple luciferases with similar self-luminous brightness and different catalytic substrates is required.

At present, luciferase that have been well researched and developed mainly includes firefly luciferase (FLuc), bacterial luciferase (Lux), luciferase extracted from renilla (*Renilla* luciferase, RLuc), luciferase extracted from oplophorus (*Oplophorus* luciferase, OLuc), and luciferase extracted from marine animal Gaussia princeps (*Gaussia* luciferase, GLuc), etc. Fluc requires cofactors such as ATP, $O_2$ and $Mg^{2+}$ and is expressed in a non-secretory way; Lux requires molecules including flavin mononucleotide (FMN), long-chain aliphatic aldehyde, oxygen and reduced nicotinamide adenine dinucleotide (NADH) for most of the fluorescent reactions; Rluc does not require ATP for Rluc luminescence, but it has week fluorescence intensity and cannot be expressed in a secretory way.

Although more than 40 bioluminescent systems have been found in nature, their exploitation is very limited.

### SUMMARY

The present disclosure is intended to solve at least one of the technical problems in the related art to some extent. To this end, an object of the present disclosure is to provide a mutant of copepoda luciferase ( also known as *Pleuromamma xiphia* luciferase (Pxluc)). By means of performing protein-directed evolution on Pxluc, the inventors have obtained a mutant having more than 2-fold increased substrate specificity to ZS26/F-CTZ and a mutant having more than 4-fold increased substrate specificity to ZS2/F-CTZ. The luciferase can be expressed in prokaryotic and eukaryotic cells, and the purification process is simple and convenient for large-scale production. The luciferase has similar luminescence as Gluc, is easy to detect, and has broad application prospects in the fields of basic scientific research, biological monitoring, biochemical diagnosis, etc.

To this end, a first aspect of the present disclosure provides a mutated luciferase. According to an embodiment of the present disclosure, the mutated luciferase has at least one of the following mutation sites based on the amino acid sequence as set forth in SEQ ID NO: 2: sites 98, 99, 100, and 101, and the mutated luciferase includes or does not include a signal peptide amino acid sequence. According to an embodiment of the present disclosure, the mutated luciferase has a stronger catalytic activity for substrates such as coelenterazine, fluorinated coelenterazine, and coelenterazine derivatives. Compared with the existing *Pleuromamma xiphia* luciferase, the mutated luciferase has a wider substrate spectrum, a stronger substrate selection specificity, and a significantly enhanced luminescence. The mutated luciferase can be used in the fields of basic science research, biological detection technology, immunodetection, biochemical detection or diagnosis where the mutated luciferase are used for a luminescence detection, and thereby have a broad application prospect.

A second aspect of the present disclosure provides a nucleic acid molecule. According to an embodiment of the present disclosure, the nucleic acid molecule encodes the mutated luciferase of the first aspect. According to an embodiment of the present disclosure, the mutant (mutated luciferase) encoded by the nucleic acid molecule has a stronger catalytic activity for substrates such as coelenterazine, fluorinated coelenterazine, and coelenterazine derivatives. Compared with existing *Pleuromamma xiphia* luciferase, the mutant has a wider substrate spectrum or a stronger substrate selection specificity, and a significantly enhanced luminescence. The mutant can be used in the fields of basic science research, biological detection technology, immunodetection, biochemical detection or diagnosis where a protein is used for a luminescence detection , and thereby have a broad application prospect.

A third aspect of the present disclosure provides an expression vector. According to an embodiment of the

present disclosure, the expression vector includes a nucleic acid molecule of the second aspect. The expression vector may include an optional control sequence, wherein the control sequence is operably linked to the nucleic acid molecule. The control sequence is one or more control sequences capable of directing expression of the nucleic acid molecule in a host. According to an embodiment of the present disclosure, the expression vector can efficiently express a protein in suitable host cells. The obtained protein has a stronger catalytic activity for substrates such as coelenterazine, fluorinated coelenterazine, and coelenterazine derivatives. Compared with the existing *Pleuromamma xiphia* luciferase, the obtained protein has a wider substrate spectrum or a stronger substrate selection specificity, and a significantly enhanced luminescence. The obtained protein can be used in the fields of basic science research, biological detection technology, immunodetection, biochemical detection or diagnosis where the protein is used for a luminescence detection, and thereby have a broad application prospect.

A fourth aspect of the present disclosure provides a recombinant cell. According to an embodiment of the present disclosure, the recombinant cell carries the nucleic acid molecule of the second aspect or the expression vector of the third aspect. The recombinant cell is obtained by transfecting or transforming the expression vector. According to an embodiment of the present disclosure, the recombinant cell can express the above-mentioned mutant with high efficiency under suitable conditions. The mutant has a stronger catalytic activity for substrates such as coelenterazine, fluorinated coelenterazine, and coelenterazine derivatives. Compared with the existing *Pleuromamma xiphia* luciferase, the mutant has a wider substrate spectrum or a stronger substrate selection specificity, and a significantly enhanced luminescence. The mutant can be used in the fields of basic science research, biological detection technology, immunodetection, biochemical detection or diagnosis where the protein is used for a luminescence detection, and thereby have a wide application prospect.

A fifth aspect of the present disclosure provides a method for producing a mutated luciferase. According to an embodiment of the present disclosure, the method includes: introducing the expression vector of the third aspect into recombinant cells; culturing and propagating the recombinant cells; and collecting product of said culturing and propagating, and extracting or purifying the mutated luciferase.

A sixth aspect of the present disclosure provides a method for detecting a nucleic acid sequence using the mutated luciferase of the first aspect. According to an embodiment of the present disclosure, the method for detecting a nucleic acid sequence includes the following steps: A) forming, by way of chemical coupling or bioconjugation or by means of fusion protein, a first mutated luciferase complex of a first specific recognition protein and the mutated luciferase of the first aspect; and forming, using a second luciferase as a signal peptide, a second mutated luciferase complex of a second specific recognition protein and the second luciferase by way of chemical coupling or bioconjugation or by means of fusion protein; B) reacting the first mutated luciferase complex with a first substrate to generate a first luminescent signal, and reacting the second luciferase complex with a second substrate to generate a second luminescent signal, wherein the first mutated luciferase complex and the second substrate do not have a significant cross-substrate reaction, and the second luciferase complex and the first substrate do not have a significant cross-substrate reaction, the first specific recognition protein recognizes and specifically binds to the first substrate, and the second specific recognition protein recognizes and specifically binds to the second substrate; and C) determining four bases A, T, G, and C for target nucleic acid sequencing by detecting fluorescence signal and signal combination of a self-luminescence system of the mutated luciferase and the second luciferase.

According to an embodiment of the present disclosure, when the mutated luciferase and the second luciferase are the same, the first substrate and the second substrate are the same; and when the mutated luciferase and the second luciferase are different, the first substrate and the second substrate are different.

According to another embodiment of the present disclosure, a method for detecting a nucleic acid sequence includes the following steps: 1) performing polymerization reaction between different bases labeled with affinity tags and having a reversible blocking modification and a template to be detected under the action of a polymerase; 2) adding a plurality of luciferase complexes in step A to couple the plurality of luciferase complexes to the different bases by specifically recognizing different affinity tags; 3) adding different substrates, determining types of the polymerized bases by detecting optical signals of the substrate or a combination thereof; and 4) adding a cleavage reagent to cleave the blocking group and linking group to prepare for the next round of polymerization.

A seventh aspect of the present disclosure provides a nucleic acid sequencing kit. According to an embodiment of the present disclosure, the kit includes a mutated luciferase of the first aspect or a luciferase complex.

An eighth aspect of the present disclosure provides a method for detecting the content of an analyte. According to an embodiment of the present disclosure, the method includes the following steps: a) forming, using the mutated luciferase of the first aspect as a signal peptide, a complex of a specific recognition protein of the analyte and the mutated luciferase by way of chemical coupling or bioconjugation or by means of fusion protein; b) contacting the analyte with the complex; c) adding a substrate for *Pleuromamma xiphia* luciferase or an analogue of the substrate to the reaction system; and d) determining the content of the analyte based on a fluorescence intensity of the reaction system detected subsequent to said adding the substrate for the *Pleuromamma xiphia* luciferase or the analogue of the substrate.

The mutant can be used as a signal protein for detecting the analyte based on the property that the mutant binds to

and reacts with the substrate. For example, the mutant is coupled or fused with a protein capable of specifically recognizing the analyte by chemical coupling or forming a fusion protein; the protein capable of specifically recognizing the analyte can bind to the analyte; the mutant can catalyze the substrate thereof and perform self-luminescence; an intensity of bioluminescence released during the process of catalyzing the substrate by the mutant is measured by a chemiluminescent microplate reader; and the content of the analyte can be determined based on a level of the activity of the mutant, the activity of the mutant being reflected by the intensity of bioluminescence.

According to a specific embodiment of the present disclosure, the analyte may be a nucleic acid. The mutant provided in the present disclosure has a stronger catalytic activity for substrates such as coelenterazine, fluorinated coelenterazine, and coelenterazine derivatives. Compared with the existing *Pleuromamma xiphia* luciferase, the mutant has a wider substrate spectrum or a stronger substrate selection specificity, and a significantly enhanced luminescence. Therefore, the method can be used for detecting the nucleic acid expression of RNA or protein such as the expression amount of an RNA or a protein, and the localization or tracing of a protein. The method for nucleic acid detection has significantly higher accuracy and sensitivity than the existing Gaussian luciferase, and the obtained results are more accurate.

In a ninth aspect of the present disclosure, the present disclosure provides a method for screening a substrate for *Pleuromamma xiphia* luciferase. The method includes: I) contacting the mutated luciferase of the first aspect with a substrate to be screened to obtain a reaction mixture; and II) determining whether the substrate to be screened is a target substrate based on whether a chemical light signal is emitted by the reaction mixture obtained in step I). Based on the characteristic that the bioluminescence can be generated by binding the mutant to a target substrate, by contacting the substrate to be screened of interest with the mutant, the bioluminescence can be emitted in the process that the substrate to be screened is catalyzed by the mutant. By means of a chemiluminescence microplate reader, it is determined whether the substrate to be screened can be catalyzed by the mutant to emit chemiluminescence, thereby determining whether the substrate to be screened is the target substrate. Therefore, the target substrate can be accurately screened out with the method of an embodiment of the present disclosure.

It is to be understood that, without departing from the scope of the present disclosure, the respective above-mentioned features of the present disclosure and the respective features specifically described below (for example, those described in the embodiments) may be combined with each other to constitute new or preferred embodiments, which are not elaborated due to the length limitation.

## BRIEF DESCRIPTION OF THE DRAWINGS

The foregoing and/or additional aspects and advantages of the present disclosure will become apparent and readily appreciated from the following description of the embodiments, in conjunction with the accompanying drawings.

FIG. 1 illustrates a plasmid profile of the wild type *Pleuromamma xiphia* luciferase prokaryotic expression plasmid pET28a-Pxluc WT containing a signal peptide;

FIG. 2 illustrates a plasmid profile of the wild type *Pleuromamma xiphia* luciferase prokaryotic expression plasmid pCold WT Pxluc containing a signal peptide;

FIG. 3 illustrates a plasmid profile of the wild type *Pleuromamma xiphia* luciferase prokaryotic expression plasmid pCold WT NS Pxluc without a signal peptide;

FIG. 4 is a graph showing the results of expression and purification of wild type *Pleuromamma xiphia* luciferase in different competent cells, with the arrows indicating bands of the protein of interest;

FIG. 5 illustrates chemical structures of substrate A (coelenterazine), substrate B (fluorinated coelenterazine), substrate C (coelenterazine derivative ZS2), and substrate D (coelenterazine derivative ZS26);

FIG. 6 illustrates results of protein level activity detection of *Pleuromamma xiphia* luciferase and Gaussian luciferase expressed in prokaryotic cells;

FIG. 7 illustrates results of protein level activity detection of the dominant mutant of *Pleuromamma xiphia* luciferase on the specific substrate ZS26/F-CTZ;

FIG. 8 illustrates results of protein level activity detection of the dominant mutant of *Pleuromamma xiphia* luciferase on the specific substrate ZS2/F-CTZ;

FIG. 9 illustrates a plasmid profile of the wild type *Pleuromamma xiphia* luciferase eukaryotic expression plasmid pEE12.4-Pxluc WT containing a signal peptide;

FIG. 10 is a graph showing the results of the expression and purification of *Pleuromamma xiphia* luciferase in eukaryotic cells, with the arrows indicating bands of the protein of interest;

FIG. 11 is a graph showing the results before and after purification of *Pleuromamma xiphia* luciferase-coupled SA protein; and

FIG. 12 illustrates results of protein level activity detection of Xiphophorus luciferase pEE12.4 Pxluc WT, mutant pEE12.4 Pxluc P26-95, coupled SA-Pxluc WT and SA Pxluc P26-95, expressed in eukaryotic cells, on the substrate-specific ZS26/F-CTZ and ZS2/F-CTZ.

## DETAILED DESCRIPTION

Embodiments of the present disclosure are described in detail below. Examples of the embodiments are illustrated in the accompanying drawings. The embodiments described below with reference to the accompanying drawing are illustrative for explaining the present disclosure, rather than being construed as limitations of the present disclosure.

Furthermore, the terms "first" and "second" are used for descriptive purposes only and are not to be construed as indicating or implying relative importance or implicitly indicating the number of technical features indicated. In this regard, a feature defined as "first" or "second" may explicitly or implicitly include at least one such feature. In the description of the present disclosure, the meaning of "plurality of" is at least two, e.g. two, three, etc., unless specifically and specifically limited otherwise.

In the field of basic research, the luciferase gene has been widely used as a reporter gene to study the expression intensity and transcriptional regulation of exogenous genes under different promoters. In the field of biological monitoring technology, luciferase can be used to detect the presence or absence of chemical contaminants. In addition, it has broad application prospects in immunodetection and biochemical diagnosis.

### Mutant

In an aspect of the present disclosure, the present disclosure provides a luciferase mutant having one or more of the following mutation sites based on the amino acid sequence as set forth in SEQ ID NO: 2: sites 98, 99, 100, and 101. The luciferase mutant includes or does not include a signal peptide amino acid sequence.

The mutants obtained by modifying the above-mentioned amino acid sequence at the mutation sites according to an embodiment of the present disclosure have a stronger catalytic activity for substrates such as coelenterazine, fluorinated coelenterazine, and coelenterazine derivatives ZS2 and ZS26. Compared with the existing *Pleuromamma xiphia* luciferase, the mutants have a wider substrate spectrum, a stronger specificity, and a significantly enhanced luminescence. The mutants can be used in the fields of basic science research, biological detection technology, immunodetection, biochemical detection or diagnosis where the mutant is used for a luminescence detection, and thereby have a broad application prospect. For example, the mutants can be used as a reporter gene for quantitative detection of DNA, RNA, transcription factors, proteins or cells, etc. The mutants can be used as a luminescence signal protein in fusion protein to quantitatively detect target small molecules, proteins, etc.

As used herein, the term "reporter gene" is a molecular biological concept referring to a type of genes that are expressed in cells, tissues/organs, or individual under specific conditions and that can produce a trait, which is readily detectable and the test material would not otherwise produce, i.e., a gene encoding a detectable protein or enzyme. A reporter gene must satisfy the following conditions in terms of genetic selection and screening detection: 1. the reporter gene has been cloned and the complete sequence thereof has been determined; 2. the expression product is not present in the recipient cells, i.e. no background, and no similar endogenous expression product exists in the transfected cells; 3. the expression product thereof can be quantitatively determined. The reporter gene can be used in the following manners, but not limited to the following manners: fusing a reporter gene with a gene expression regulatory sequence to form a chimeric gene, or fusing a reporter gene with other genes of interest, allowing the nucleic acid to express under the control of the regulatory sequence, detecting the expression regulation of the gene of interest based on the expression product thereof, and studying the nucleic acid.

As used herein, the term "nucleic acid expression" may refer to the expression of DNA as RNA, or the expression of DNA as a protein via RNA, or the expression of RNA as a protein. That is, the product obtained after expression of the nucleic acid herein can be either an RNA or a protein.

As used herein, "chemiluminescence", also referred to as cold light, is optical radiation produced by a chemical reaction in the absence of any excitation by light, heat, electric field, or the like. Chemiluminescence is also found in living systems, referred to as bioluminescence, e.g., light emitted by firefly, certain bacteria or fungi, protozoa, helminths, and crustaceans. In the present disclosure, the mutants may undergo self-luminescence, i.e., chemiluminescence.

According to some specific embodiments of the present disclosure, the above-mentioned mutant may further include at least one of the following additional technical features.

According to some specific embodiments of the present disclosure, the gene sequence of wild type *Pleuromamma xiphia* luciferase (WT Pxluc) containing a signal peptide is as set forth in SEQ ID NO: 1, and the encoded amino acid thereof is as set forth in SEQ ID NO: 2, in which amino acids at sites 1 to 17 are the signal peptide (in bold); and the gene sequence of the wild type *Pleuromamma xiphia* luciferase without a signal peptide (WT no signal peptide Pxluc: WT-NS Pxluc) is as set forth in SEQ ID NO: 3, and the encoded amino acid thereof is as set forth in SEQ ID NO: 4. The sequences as set forth in SEQ ID NO: 1 to SEQ ID NO: 4 are as follows:

ATGTATATAAAAGTTTGGTTTGGTCTGGCTTGTCTTTCATTGGTTCTGGCC
CAACCAACTGAAAACAAGCAGGAGTCTCATATTGTAGATTCAGATCTTGATGGCGA
CCGTGGTAGGAAGTTGCCCGGAAAAAAGCTTCCTATAGAAGTACTCAAAATCATG
GAAGCCAATGCCAGGAGAGCTGGTTGCACTAGAGGATGTCTCATATGTCTTTCAAA
AATCAAGTGTACAGCCAAAATGAAGCGATACATTCCAGGGAGATGTCATACTTATG
AAGGAGATAAATCTATTGGACAGGGAGGCATAGGTGGCCCTATTGTTGATATTCCTG
AAATTATTGGATTCAAGAACATGGAACCCATGGATCAGTTCATCGCACAAGTTGAT
CTGTGCGCCGACTGTACAACTGGGTGCCTGAAAGGCCTTGCTAATGTTAGGTGCA
ATGACTTGCTGAAGAAATGGCTGCCTGACAGATGTGCTGGTTTTGCCGACAAAATT
CAAAATGAAGTGGATAGTATCAAGGGCATGGCTGGGGATCGC (SEQ ID NO: 1)

**MYIKVWFGLACLSLVLA**QPTENKQESHIVDSDLDGDRGRKLPGKKLPIEV
LKIMEANARRAGCTRGCLICLSKIKCTAKMKRYIPGRCHTYEGDKSI**G**QGGIGGPIVDI
PEIIGFKNMEPMDQFIAQVDLCADCTTGCLKGLANVRCNDLLKKWLPDRCAGFADKI
QNEVDSIKGMAGDR (SEQ ID NO: 2)

CAACCAACTGAAAACAAGCAGGAGTCTCATATTGTAGATTCAGATCTTG
ATGGCGACCGTGGTAGGAAGTTGCCCGGAAAAAAGCTTCCTATAGAAGTACTCAA
AATCATGGAAGCCAATGCCAGGAGAGCTGGTTGCACTAGAGGATGTCTCATATGTC
TTTCAAAAATCAAGTGTACAGCCAAAATGAAGCGATACATTCCAGGGAGATGTCAT
ACTTATGAAGGAGATAAATCTATTGGACAGGGAGGCATAGGTGGCCCTATTGTTGA
TATTCCTGAAATTATTGGATTCAAGAACATGGAACCCATGGATCAGTTCATCGCACA
AGTTGATCTGTGCGCCGACTGTACAACTGGGTGCCTGAAAGGCCTTGCTAATGTTA
GGTGCAATGACTTGCTGAAGAAATGGCTGCCTGACAGATGTGCTGGTTTTGCCGA
CAAAATTCAAAATGAAGTGGATAGTATCAAGGGCATGGCTGGGGATCGC (SEQ ID NO: 3)

QPTENKQESHIVDSDLDGDRGRKLPGKKLPIEVLKIMEANARRAGCTRGC
LICLSKIKCTAKMKRYIPGRCHTYEGDKSIGQGGIGGPIVDIPEIIGFKNMEPMDQFIAQ
VDLCADCTTGCLKGLANVRCNDLLKKWLPDRCAGFADKIQNEVDSIKGMAGDR
(SEQ ID NO: 4)

According to some specific embodiments of the present disclosure, the mutant has one or more of the following mutations (1) to (4) based on the amino acid sequence as set forth in SEQ ID NO: 2:

(1) G at site 98 is mutated to L, P, Q, S, or T;
(2) Q at site 99 is mutated to R, W, I, Y, A, L, F, V, P, E, or M;
(3) G at site 100 is mutated to S, Q, R, W, T, A, or L; and
(4) G at site 101 is mutated to F, R, S, C, Y, L, I, K, V, or P.

According to specific embodiments of the present disclosure, the mutated luciferase has one or two of the following mutation sites based on the amino acid sequence as set forth in SEQ ID NO: 2: sites 98, 99, 100 and 101, and the mutated luciferase includes or does not include a signal peptide amino acid sequence.
According to further specific embodiments of the present disclosure, the mutant has one or two of the following mutations (1) to (4) based on the amino acid sequence as set forth in SEQ ID NO: 2:

(1) G at site 98 is mutated to L, P, Q, S, or T;
(2) Q at site 99 is mutated to R, W, I, Y, A, L, F, V, P, E, or M;
(3) G at site 100 is mutated to S, Q, R, W, T, A, or L; and
(4) G at site 101 is mutated to F, R, S, C, Y, L, I, K, V, or P.

According to some specific embodiments of the present disclosure, the mutant has the following mutations based on the amino acid sequence as set forth in SEQ ID NO: 2:

1) G at site 98 is mutated to L, and Q at site 99 is mutated to R; or
2) G at site 98 is mutated to P; or
3) G at site 98 is mutated to Q; or
4) G at site 98 is mutated to S, and Q at site 99 is mutated to W; or
5) Q at site 98 is mutated to I; or
6) Q at site 99 is mutated to Y; or
7) Q at site 99 is mutated to A; or
8) Q at site 99 is mutated to L; or
9) Q at site 99 is mutated to F; or
10) G at site 98 is mutated to L, and Q at site 99 is mutated to V; or
11) G at site 98 is mutated to T, and Q at site 99 is mutated to P; or
12) G at site 98 is mutated to L, and Q at site 99 is mutated to E; or
13) Q at site 99 is mutated to M, and G at site 100 is mutated to S; or
14) G at site 100 is mutated to Q, and G at site 101 is mutated to F; or
15) G at site 100 is mutated to R, and G at site 101 is mutated to R; or
16) G at site 100 is mutated to W, and G at site 101 is mutated to F; or
17) G at site 100 is mutated to S; or
18) G at site 100 is mutated to T, and G at site 101 is mutated to S; or
19) G at site 100 is mutated to R, and G at site 101 is mutated to C; or
20) G at site 100 is mutated to A, and G at site 101 is mutated to R; or
21) G at site 100 is mutated to L, and G at site 101 is mutated to Y; or
22) G at site 100 is mutated to S, and G at site 101 is mutated to L; or
23) G mutation at site 101 is I; or
24) G at site 100 is mutated to L, and G at site 101 is mutated to K; or
25) G at site 100 is mutated to T; or
26) G at site 100 is mutated to A, and G at site 101 is mutated to V; or
27) G at site 100 is mutated to A, and G at site 101 is mutated to P.

According to some specific embodiments of the present disclosure, when the amino acid sequence as set forth in SEQ ID NO: 2 has the above-mentioned mutation, the obtained protein has a relatively stronger catalytic activity for substrates such as coelenterazine, fluorinated coelenterazine, and coelenterazine derivatives. Compared with the existing *Pleuromamma xiphia* luciferase, the protein has a wider substrate spectrum and significantly enhanced luminescence. The protein has a significantly improved detection accuracy in practical applications, and can be used in the fields of basic science research, biological detection technology, immunodetection, biochemical detection or diagnosis where the protein is used for a luminescence detection, and thereby has a broad application prospect.
According to some specific embodiments of the present disclosure, the mutated luciferase does not contain a

signal peptide sequence.

According to some specific embodiments of the present disclosure, the mutated luciferase may be a luciferase having a mutation at one or more of sites 98, 99, 100, and 101 based on the amino acid sequence as set forth in SEQ ID NO: 2 (e.g., the mutation may be at any one of the four mutation sites, or at two of the four mutation sites in combination, or at three of the four mutation sites in combination, or at all of the four mutation sites).

According to some specific embodiments of the present disclosure, the mutated luciferase may also have one or more of the mutation sites 98, 99, 100, and 101 based on the amino acid sequence as set forth in SEQ ID NO: 2, and the mutated luciferase does not contain a signal peptide amino acid sequence.

The amino acids at sites 1 to 17 of the amino acid sequence as set forth in SEQ ID NO: 2 are a signal peptide. The inventors found that, as long as one or more of sites 98, 99, 100, and 101 (these sites are in the SEQ ID NO: 2 sequence) are mutated, the mutated luciferase obtained based on either the complete sequence of SEQ ID NO: 2 containing the signal peptide or the sequence without the signal peptide can have a stronger catalytic activity for substrates such as coelenterazine, fluorinated coelenterazine, and coelenterazine derivative ZS2, and has a wider substrate spectrum, a stronger specificity, and a significantly enhanced luminescence compared with the existing *Pleuromamma xiphia* luciferase. According to some specific embodiments of the present disclosure, the mutant is a non-secreted protein or a secreted protein.

According to some specific embodiments of the present disclosure, the present disclosure provides a nucleic acid molecule encoding the above-mentioned mutant.

It should be noted that, with respect to the nucleic acid mentioned in the present specification and claims, those skilled in the art can understand that the nucleic acid includes any one of or both of complementary duplexes. For convenience, in this specification and claims, while in most cases only one strand is shown, in fact, the other strand complementary thereto is also disclosed. In addition, the nucleic acid sequences of the present disclosure include DNA forms or RNA forms, one of which is disclosed, meaning that the other is also disclosed.

In another aspect of the present disclosure, the present disclosure provides an expression vector including the above-mentioned nucleic acid molecule. The expression vector herein is not limited a specific type, as long as the corresponding mutant is capable of replicative expression in a host cell. The expression vector may include an optional control sequence, wherein the control sequence is operably linked to the nucleic acid molecule. The control sequence is one or more control sequences capable of directing expression of the nucleic acid molecule in a host. According to some specific embodiments of the present disclosure, the expression vector can efficiently express a protein in a suitable host cell, and the obtained protein has a stronger catalytic activity for substrates such as coelenterazine, fluorinated coelenterazine, and coelenterazine derivatives. Compared with the existing *Pleuromamma xiphia* luciferase, the protein has a wider substrate spectrum, a stronger specificity, and a significantly enhanced luminescence. The obtained protein can be used in the fields of basic science research, biological detection technology, immunodetection, biochemical detection or diagnosis where the protein is used for a luminescence detection, and thereby have a broad application prospect.

In a further aspect, the present disclosure provides a recombinant cell carrying a nucleic acid molecule, expression vector, or mutant as described above. The recombinant cell is obtained by transfecting or transforming the expression vector. According to some specific embodiments of the present disclosure, the recombinant cell can express the above-mentioned mutant under suitable conditions with high efficiency. The mutant has a stronger catalytic activity for substrates such as coelenterazine, fluorinated coelenterazine and coelenterazine derivatives. Compared with the existing *Pleuromamma xiphia* luciferase, the mutant has a wider substrate spectrum, a stronger specificity and a significantly enhanced luminescence. The mutant can be used in the fields of basic science research, biological detection technology, immunodetection, biochemical detection or diagnosis where the protein is used for a luminescence detection, and thereby have a broad application prospect.

According to some specific embodiments of the present disclosure, the above-mentioned recombinant cell may further include at least one of the following additional technical features.

According to some specific embodiments of the present disclosure, the recombinant cell is *Escherichia coli,* yeast, or mammalian cell. According to some specific embodiments of the present disclosure, the recombinant cell is not specifically limited. Any cell capable of expressing the mutant in a nucleic acid or vector (e.g., plasmid), for example, yeast cells, bacteria, or mammalian cells such as human embryonic kidney cells, can be used as the recombinant cells.

According to some specific embodiments of the present disclosure, the recombinant cells do not include animal germ cells, fertilized eggs, or embryonic stem cells.

According to some specific embodiments of the present disclosure, the present disclosure provides a method for producing a mutated luciferase. The method includes: introducing the expression vector described above into recombinant cells; culturing and propagating the recombinant cells; and collecting product of said culturing and propagating, and extracting or purifying the mutated luciferase. According to some specific embodiments of the present disclosure, the present disclosure provides the use of the mutated luciferase as described above and a corresponding substrate in the detection of a nucleic acid sequence.

It should be noted that in the present disclosure, "mutated luciferase", "luciferase mutant", and "mutant" can be equivalently replaced with each other.

**Kit and use for prepared kit**

In an aspect of the present disclosure, the present disclosure provides a kit for detecting a content of an analyte. The kit includes the luciferase mutant as described above. A specific recognition protein of the analyte is suitable for forming a complex with the mutant.

According to some specific embodiments of the present disclosure, the kit further includes a substrate for *Pleuromamma xiphia* luciferase or an analogue of the substrate.

According to some specific embodiments of the present disclosure, the substrate is selected from at least one of coelenterazine, fluorinated coelenterazine, or coelenterazine derivative.

According to some specific embodiments of the present disclosure, coelenterazine derivative is selected from coelenterazine derivative ZS2 or coelenterazine derivative ZS26.

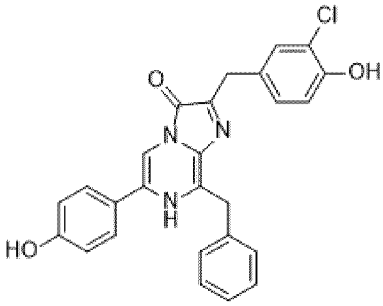

ZS2

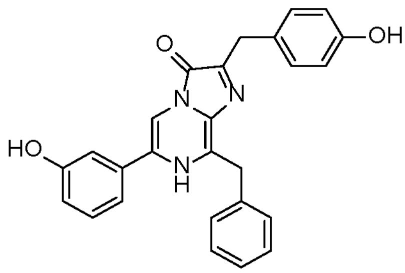

ZS26

According to some specific embodiments of the present disclosure, the kit further includes a specific recognition protein for the analyte.

The present disclosure provides a nucleic acid sequencing kit. The kit includes the mutated luciferase as described above.

In a further aspect of the present disclosure, the present disclosure provides the use of the mutant as described above in the preparation of a kit for detecting a content of an analyte, and a specific recognition protein of the analyte is suitable for forming a complex with the mutant. Based on the characteristic that the mutant reacts with the substrate, the mutant can be used as a signal protein to detect the analyte. For example, by means of chemical coupling or forming a fusion protein, the mutant is coupled or fused with a protein capable of specifically recognizing the analyte. When the analyte, the mutant, and the substrate are in the same system, the protein capable of specifically recognizing the analyte can bind to the analyte. The mutant can catalyze the substrate thereof and perform self-luminescence. The intensity of the bioluminescence released by the mutant during the process of catalyzing the substrate thereof is determined by a chemiluminescent microplate reader. The intensity of the bioluminescence can reflect the activity of the mutant, and the content of the analyte can be determined based on the level of the activity. Before the substrate binds to the mutant, it is necessary to wash off the non-specific binding protein in the system, to exclude the interference of other factors on the detection result. Thus, the mutant can be used to prepare a kit and accurately detect the content of the analyte.

According to some specific embodiments of the present disclosure, the above-mentioned use may further include at least one of the following additional technical features.

According to some specific embodiments of the present disclosure, the kit further includes a substrate for *Pleuromamma xiphia* luciferase or an analogue of the substrate.

According to some specific embodiments of the present disclosure, the substrate is selected from at least one of coelenterazine, fluorinated coelenterazine, or coelenterazine derivative.

According to some specific embodiments of the present disclosure, the substrate is not specifically limited. The substances that can chemically react with the mutant shall fall within the scope of the substrate (but not limited to chemiluminescence reaction). A person skilled in the art can change different substrates according to experimental requirements.

According to some specific embodiments of the present disclosure, coelenterazine derivative is selected from coelenterazine derivative ZS2 or coelenterazine derivative ZS26.

According to some specific embodiments of the present disclosure, the kit further includes a specific recognition protein for the analyte.

**Methods**

In an aspect of the present disclosure, the present disclosure provides a method for detecting a content of a substance. The method includes the following steps:

i) forming a complex between the analyte-specific recognition protein and the mutant as described above;
ii) contacting an analyte with the complex;
iii) adding a substrate for *Pleuromamma xiphia* luciferase or an analogue of the substrate to the reaction system;
iv) determining a content of an analyte based on fluorescence intensity of the reaction system subsequent to said adding the substrate for *Pleuromamma xiphia* luciferase or the analogue of the substrate.

According to some specific embodiments of the present disclosure, the present disclosure provides a method for detecting a content of an analyte. The method includes the following steps:

a) forming, using the above-mentioned mutated luciferase as a signal protein, a complex of a specific recognition protein of the analyte and the mutated luciferase by way of chemical coupling or bioconjugation or by means of fusion protein;
b) contacting the analyte with the complex;
c) adding a substrate for *Pleuromamma xiphia* luciferase or an analogue of the substrate to the reaction system;
d) determining the content of the analyte based on a fluorescence intensity of the reaction system detected subsequent to said adding the substrate for the *Pleuromamma xiphia* luciferase or the analogue of the substrate.

According to some specific embodiments of the present disclosure, the substrate is selected from at least one of coelenterazine, fluorinated coelenterazine, or coelenterazine derivative.
According to some specific embodiments of the present disclosure, coelenterazine derivative is selected from coelenterazine derivative ZS2 or coelenterazine derivative ZS26.
In yet another aspect of the present disclosure, the present disclosure provides a method for screening a substrate for *Pleuromamma xiphia* luciferase. The method includes:

I) contacting the above-mentioned mutant with a substrate to be screened to obtain a reaction mixture; and
II) determining, based on whether the reaction mixture obtained in step I) emits a chemical light signal, whether the substrate to be screened is a target substrate.

According to some specific embodiments of the present disclosure, the reaction mixture obtained in step I) emits a chemical light signal indicative that the substrate to be screened is a target substrate.
The present disclosure is described with reference to specific examples. These examples are intended to be illustrative only and not limiting in any way.

**Example 1: Design and construction of plasmid for wild-type *Pleuromamma xiphia* luciferase to be expressed in prokaryotic cells**

In the present example, wild type *Pleuromamma xiphia* luciferase with and without a signal peptide were constructed to evaluate the effects of the signal peptide on the wild type *Pleuromamma xiphia* luciferase.
The gene sequence of the wild type *Pleuromamma xiphia* luciferase (WT Pxluc) containing a signal peptide is as set forth in SEQ ID NO: 1, and the encoded amino acid thereof is as set forth in SEQ ID NO: 2, in which amino acids at sites 1 to 17 are the signal peptide (in bold); and the gene sequence of the wild type *Pleuromamma xiphia* luciferase without a signal peptide (WT no signal peptide Pxluc: WT-NS Pxluc) is as set forth in SEQ ID NO: 3, and the encoded amino acid thereof is as set forth in SEQ ID NO: 4.
The gene sequence, as set forth in SEQ ID NO: 5, of *Pleuromamma xiphia* luciferase containing a signal peptide for pET28a vector (pET28a Pxluc WT) was synthesized by means of total gene synthesis technology (Sangon Biotech). The plasmid profile is illustrated in FIG. 1, which was fused with a purification tag containing six histidine (6 x His) at the C-terminal to facilitate protein purification. The cleavage sites at both ends were BamHI and NotI.
The gene sequence, as set forth in SEQ ID NO: 6, of *Pleuromamma xiphia* luciferase containing a signal peptide for pCold vector was synthesized by means of PCR technology and fused with a purification tag containing six histidine (6 x His) at the C-terminal to facilitate protein purification. The gene sequence, as set forth in SEQ ID NO: 7, of *Pleuromamma xiphia* luciferase without the signal peptide for pCold vector was synthesized by means of PCR technology and fused with a purification tag containing six histidine (6 x His) at the C-terminal to facilitate protein purification.

ATGTATATAAAAGTTTGGTTTGGTCTGGCTTGTCTTTCATTGGTTCTGGCCCAACCAACTGAAAACAAGCAGGAGTCTCATATTGTAGATTCAGATCTTGATGGCGACCGTGGTAGGAAGTTGCCCGGAAAAAAGCTTCCTATAGAAGTACTCAAAATCATGGAAGCCAATGCCAGGAGAGCTGGTTGCACTAGAGGATGTCTCATATGTCTTTCAAAAATCAAGTGTACAGCCAAAATGAAGCGATACATTCCAGGGAGATGTCATACTTATGAAGGAGATAAATCTATTGGACAGGGAGGCATAGGTGGCCCTATTGTTGATATTCCTGAAATTATTGGATTCAAGAACATGGAACCCATGGATCAGTTCATCGCACAAGTTGATCTGTGCGCCGACTGTACAACTGGGTGCCTGAAAGGCCTTGCTAATGTTAGGTGCAATGACTTGCTGAAGAAATGGCTGCCTGACAGATGTGCTGGTTTTGCCGACAAAATTCAAAATGAAGTGGATAGTATCAAGGGCATGGCTGGGGATCGC (SEQ ID NO: 1)

**MYIKVWFGLACLSLVLA**QPTENKQESHIVDSDLDGDRGRKLPGKKLPIEVLKIMEANARRAGCTRGCLICLSKIKCTAKMKRYIPGRCHTYEGDKSI**G**QGGIGGPIVDIPEIIGFKNMEPMDQFIAQVDLCADCTTGCLKGLANVRCNDLLKKWLPDRCAGFADKIQNEVDSIKGMAGDR (SEQ ID NO: 2)

CAACCAACTGAAAACAAGCAGGAGTCTCATATTGTAGATTCAGATCTTGATGGCGACCGTGGTAGGAAGTTGCCCGGAAAAAAGCTTCCTATAGAAGTACTCAAAATCATGGAAGCCAATGCCAGGAGAGCTGGTTGCACTAGAGGATGTCTCATATGTCTTTCAAAAATCAAGTGTACAGCCAAAATGAAGCGATACATTCCAGGGAGATGTCATACTTATGAAGGAGATAAATCTATTGGACAGGGAGGCATAGGTGGCCCTATTGTTGATATTCCTGAAATTATTGGATTCAAGAACATGGAACCCATGGATCAGTTCATCGCACAAGTTGATCTGTGCGCCGACTGTACAACTGGGTGCCTGAAAGGCCTTGCTAATGTTAGGTGCAATGACTTGCTGAAGAAATGGCTGCCTGACAGATGTGCTGGTTTTGCCGACAAAATTCAAAATGAAGTGGATAGTATCAAGGGCATGGCTGGGGATCGC (SEQ ID NO: 3)

QPTENKQESHIVDSDLDGDRGRKLPGKKLPIEVLKIMEANARRAGCTRGCLICLSKIKCTAKMKRYIPGRCHTYEGDKSIGQGGIGGPIVDIPEIIGFKNMEPMDQFIAQVDLCADCTTGCLKGLANVRCNDLLKKWLPDRCAGFADKIQNEVDSIKGMAGDR (SEQ ID NO: 4)

aagcttgccgccaccATGTATATAAAAGTTTGGTTTGGTCTGGCTTGTCTTTCATTGGTTCTGGCCCAACCAACTGAAAACAAGCAGGAGTCTCATATTGTAGATTCAGATCTTGATGGCGACCGTGGTAGGAAGTTGCCCGGAAAAAAGCTTCCTATAGAAGTACTCAAAATCATGGAAGCCAATGCCAGGAGAGCTGGTTGCACTAGAGGATGTCTCATATGTCTTTCAAAAATCAAGTGTACAGCCAAAATGAAGCGATACATTCCAGGGAGATGTCATACTTATGAAGGAGATAAATCTATTGGACAGGGAGGCATAGGTGGCCCTATTGTTGATATTCCTGAAATTATTGGATTCAAGAACATGGAACCCATGGATCAGTTCATCGCACAAGTTGATCTGTGCGCCGACTGTACAACTGGGTGCCTGAAAGGCCTTGCTAATGTTAGGTGCAATGACTTGCTGAAGAAATGGCTGCCTGACAGATGTGCTGGTTTTGCCGACAAAATTCAAAATGAAGTGGATAGTATCAAGGGCATGGCTGGGGATCGCggtggcagtgaaaatctgtattttcagagcggcggccatcatcatcatcatcatggcagctgatgagaattc (SEQ ID NO: 5)

caccatgaatcacaaagtgcatatgATGTATATAAAAGTTTGGTTTGGTCTGGCTTGTCTTTCATTGGTTCTGGCCCAACCAACTGAAAACAAGCAGGAGTCTCATATTGTAGATTCAGATCTTGATGGCGACCGTGGTAGGAAGTTGCCCGGAAAAAAGCTTCCTATAGAAGTACTCAAAATCATGGAAGCCAATGCCAGGAGAGCTGGTTGCACTAGAGGATGTCTCATATGTCTTTCAAAAATCAAGTGTACAGCCAAAATGAAGCGATACATTCCAGGGAGATGTCATACTTATGAAGGAGATAAATCTATTGGACAGGGAGGCATAGGTGGCCCTATTGTTGATATTCCTGAAATTATTGGATTCAAGAACATGGAACCCATGGATCAGTTCATCGCACAAGTTGATCTGTGCGCCGACTGTACAACTGGGTGCCTGAAAGGCCTTGCTAATGTTAGGTGCAATGACTTGCTGAAGAAATGGCTGCCTGACAGATGTGCTGGTTTTGCCGACAAAATTCAAAATGAAGTGGATAGTATCAAGGGCATGGCTGGGGATCGCggtggcagtgagaacctgtac (SEQ ID NO: 6)

catgaatcacaaagtgcatatgCAACCAACTGAAAACAAGCAGGAGTCTCATATTGTAGATTCAGATCTTGATGGCGACCGTGGTAGGAAGTTGCCCGGAAAAAAGCTTCCTATAGAAGTACTCAAAATCATGGAAGCCAATGCCAGGAGAGCTGGTTGCACTAGAGGATGTCTCATATGTCTTTCAAAAATCAAGTGTACAGCCAAAATGAAGCGATACATTCCAGGGAGATGTCATACTTATGAAGGAGATAAATCTATTGGACAGGGAGGCATAGGTGGCCCTATTGTTGATATTCCTGAAATTATTGGATTCAAGAACATGGAACCCATGGATCAGTTCATCGCACAAGTTGATCTGTGCGCCGACTGTACAACTGGGTGCCTGAAAGGCCTTGCTAATGTTAGGTGCAATGACTTGCTGAAGAAATGGCTGCCTGACAGATGTGCTGGTTTTGCCGACAAAATTCAAAATGAAGTGGATAGTATCAAGGGCATGGCTGGGGATCGCggtggcagtgagaacctgta (SEQ ID NO: 7)

1. Obtaining inserted fragment

Whole gene synthetic wild type *Pleuromamma xiphia* luciferase containing a signal peptide (pET28a Pxluc WT) was dissolved in deionized water and diluted to a concentration of 10 ng/μl, as template DNA. By using the KOD FXD Neo enzyme, PCR reaction system was prepared and PCR reaction was performed according to the manual thereof, to prepare the inserted fragment.

Table 1 lists the sequences of primers used for the PCR reaction of the wild type *Pleuromamma xiphia* luciferase containing a signal peptide (WT-Pxluc) and the wild type *Pleuromamma xiphia* luciferase without a signal peptide (WT-NS Pxluc). The PCR reaction system is shown in Table 2. The reaction conditions are shown in Table 3, and the number of PCR cycles is 30.

[Table 1]

| Name | Sequence |
|---|---|
| Forward Primer insert-F1 (WT Pxluc) | 5'-CACCATGAATCACAAAGTGCATATGTATATAAAAGTTTGG-3' (SEQ ID NO: 8) |
| Forward Primer insert-F2 (WT-NS Pxluc) | 5'-CATGAATCACAAAGTGCATATGCAACCAACTGAAAACAAGCAGG-3' (SEQ ID NO: 9) |
| Reverse Primer insert-R | 5'-GTACAGGTTCTCACTGCCACCGCGATCCCCAGCCATGCCC-3' (SEQ ID NO: 10) |

[Table 2]

| Components | Volume (μL) |
|---|---|
| 2x KOD FX Neo PCR buffer solution | 25 |
| 2 mM dNTPs | 10 |
| 10 μM Primer insert-F | 1.5 |

(continued)

| Components | Volume (μL) |
|---|---|
| 10 μM Primer insert-R | 1.5 |
| Template DNA (10 ng/μL) | 2.5 |
| KOD FX Neo | 1 |
| PCR grade water | 8.5 |
| Total Volume | 50 |

[Table 3]

| Phase | Temperature | Time | Number of cycles |
|---|---|---|---|
| Pre-denaturation | 94°C | 2 min | 1 |
| Degeneration | 98°C | 10 s | 30 |
| Annealing | 60°C | 30 s | |
| Extension | 68°C | 40 s | |
| Extension | 68°C | 5 min | 1 |

The template was digested by adding 0.5 μL of DpnI enzyme to the reaction system and incubating at 37°C for 3 h, and then the products of about 586 bp and 531 bp were recovered as inserted fragments, i.e., inserts. The gel-recovered fragment of 586 bp was the inserted fragment containing a signal peptide, and the gel-recovered fragment of 531 bp was the inserted fragment without a signal peptide.

2. Obtaining linearized vector

Using the pCold vector as a template, the vector was linearized by means of PCR to facilitate recombination with the inserted fragment. By using KOD FX neo enzymes, PCR reaction system was prepared and PCR reaction was performed according to the manual thereof.

The sequences of primers used in the PCR reaction are shown in Table 4. The PCR reaction system is shown in Table 5. The reaction conditions are shown in Table 6, and the number of PCR cycles is 30.

[Table 4]

| Name | Sequence |
|---|---|
| Forward Primer vector-F1 | 5'-GGGCATGGCTGGGGATCGCGGTGGCAGTGAGAACCTGTAC-3' (SEQ ID NO: 11) |
| Reverse Primer vector-R1 (WT Pxluc) | 5'-CCAAACTTTTATATACATATGCACTTTGTGATTCATGGTG-3' (SEQ ID NO: 12) |
| Reverse Primer vector-R2 (WT-NS Pxluc) | 5'-CCTGCTTGTTTTCAGTTGGTTGCATATGCACTTTGTGATTCATG-3' (SEQ ID NO: 13) |

[Table 5]

| Components | Volume (μL) |
|---|---|
| 2x KOD FX Neo PCR buffer solution | 25 |

(continued)

| Components | Volume (μL) |
|---|---|
| 2 mM dNTPs | 10 |
| 10 μM Primer vector-F | 1.5 |
| 10 μM Primer vector-R | 1.5 |
| pCold (10 ng/μL) | 2.5 |
| KOD FX Neo enzyme | 1 |
| PCR grade water | 8.5 |
| Total Volume | 50 |

[Table 6]

| Phase | Temperature | Time | Number of cycles |
|---|---|---|---|
| Pre-denaturation | 94°C | 2 min | 1 |
| Degeneration | 98°C | 10 s | 30 |
| Annealing | 58°C | 30 s | |
| Extension | 68°C | 5 min | |
| Extension | 68°C | 5 min | 1 |

The template was digested by adding 0.5 μL of DpnI enzyme to the reaction system and incubating at 37 °C for 3 h, and then the product of about 4433 bp was recovered as a linearized vector.

Insert and vector were reconstituted using the In-Fusion Cloning kit (TAKARA) according to the reaction system shown in Table 7 and incubated at 50 °C for 15 min.

[Table 7]

| Components | Volume (μL) |
|---|---|
| Inserted fragment (10 ng/μL) | 5 |
| Linear vector (10 ng/μL) | 3 |
| Premix | 2 |
| Total Volume | 10 |

2.5 μL of the above reaction product was taken and transformed into DH5α competent cells and spread onto plates containing ampicillin resistance with a final concentration of 100 μg/mL. On the next day, a single clone was picked from the plates, and the plasmids were extracted for sequencing to ensure that the target fragment was correctly inserted into the vector. The obtained plasmid was the wild *Pleuromamma xiphia* luciferase containing a signal peptide, i.e., pCold-WT Pxluc (FIG. 2); and the wild *Pleuromamma xiphia* luciferase without a signal peptide, i.e., pCold WT NS Pxluc (FIG. 3), which were consistent with the experimental expectation.

**Example 2: Prokaryotic expression, purification, and protein level activity assay of *Pleuromamma xiphia* luciferase**

1. Prokaryotic expression of *Pleuromamma xiphia* luciferase

The expression plasmid pET28a-Pxluc WT was transformed into BL21 (DE3) competent cells, and the expression plasmids pCold WT Pxluc and pCold WT-NS Pxluc were transformed into OrigamiB (DE3) chemically competent cells (EC1020S, WEIDI), respectively. The cells were plated, and a single colony was picked from the plate and incubated overnight at 37 °C. On the next day, the cells were diluted at a ratio of 1: 100 and transferred to 300 ml of fresh LB medium containing kanamycin (50 μg/ml) or ampicillin (100 μg/ml), respectively. The cells were incubated at 37°C with shaking at 200 rpm until OD600 was about 0.5 to 0.6, and cooled on ice for 1 h. The inducer IPTG was added at a final concentration of

1 mM and induced overnight at 16°C.

2. Purification of *Pleuromamma xiphia* luciferase

The induced bacterial liquid precipitate was collected by centrifugation at a speed of 8,000 rpm/min for 10 min, and added with 30 ml of binding buffer solution (50 mM Tris, 250 mM NaCl, pH 8.0) and 300 μL of lysozyme. The mixture was lysed on ice for 30 min, disrupted by ultrasonic wave (turning on for 2 seconds, turning off for 3 seconds, 60% power) for 30 min, and the supernatant (cell lysate) and precipitate were separated by centrifugation at 12,000 rpm for 30 min at 4°C.

2 mL of HisTrap FF filler was added to the manual column (purchased from Sangon Biotech, model F506607-0001# affinity chromatography column empty column) and the equilibrated filler was washed with 30 ml of binding buffer solution. Approximately 30 ml of filtered cell lysate was then added. After washing 10 times (10 ml/time) with rinsing solution (50 mM Tris, pH 8.0, 250 mM NaCl, 10 mM imidazole) and then protein was eluted 4 to 5 times with 500 μL of eluent (50 mM Tris, pH 8.0, 250 mM NaCl, 300 mM imidazole), and the eluted protein was collected. Dialysis was performed overnight at 4°C Protein concentration and purity profiles were determined by the BCA quantification kit method and the SDS-PAGE method.

The protein obtained after elution was determined by the SDS-PAGE method, and the purification results are shown in FIG. 4. FIG. 4 indicates that the target protein was successfully expressed in BL21 (DE3) cells transformed with pET28a-Pxluc WT plasmid, as well as in origamiB (DE3) cells transformed with expression plasmids pCold WT Pxluc and pCold WT-NS Pxluc.

3. Protein activity assay for *Pleuromamma xiphia* luciferase

The concentration of protein was accurately determined using a BCA quantification kit (Thermo Scientific™ Pierce™ BCA Protein Assay Kit), and the above purified luciferase was diluted with diluent (50 mM Tris-HCl pH 8.0, 100 mM NaCl, 0.1% (v/v) Tween-20). The protein expressed by the pET28a-Pxluc WT plasmid was diluted to 12 μg/ml, and the proteins expressed by the pCold WT Pxluc plasmid and pCold WT-NS Pxluc plasmid were diluted to 1 μg/ml. 10 μL of each protein was added to a black 96-well plate, which was then added with 90 μL of the respective substrates, coelenterazine (purchased from Baiaolaibo), fluorinated coelenterazine, and coelenterazine derivatives ZS2, ZS26 (the structural formula of each compound is shown in FIG. 5) that were diluted to 100 μM with the same solution, and the luminescence intensity was read from the luminescence module with a microplate reader. The activity assay results of *Pleuromamma xiphia* luciferase and coelenterazine (CTZ) were shown in FIG. 6. Gluc expressed in OrigamiB (DE3) competent cells was known to have good luminescence activity, and thus it was used as a control group. The results in FIG. 6 show that OrigamiB (DE3) competent cells containing plasmid pCold WT NS Pxluc had the highest luminescence value, which is comparable to that of OrigamiB (DE3) competent cells containing plasmid pCold Gluc. Therefore, the luminescence activity of *Pleuromamma xiphia* luciferase without a signal peptide to coelenterazine is similar to that of Gluc to coelenterazine. Compared with *Pleuromamma xiphia* luciferase containing a signal peptide, *Pleuromamma xiphia* luciferase without a signal peptide had higher activity. Subsequent experiments were performed with *Pleuromamma xiphia* luciferase without a signal peptide.

### Example 3: Design and construction of library of *Pleuromamma xiphia* luciferase mutants

Using pCold WT-NS Pxluc plasmid sequence as a template, site-directed saturation mutant libraries (specific amino acid was mutated to any other amino acid) of sites G98, Q99, G100, and G101 on sequence as set forth in SEQ ID NO: 2 were synthesized by PCR technique. The mutant library 1 was a site-directed saturation mutant library at amino acid position G98 (L1). The mutant library 2 was a site-directed saturation mutant library at site Q99 (L2). The mutant library 3 was a site-directed saturation mutant library at amino acid position G100 (L3). The mutant library 4 was a site-directed saturation mutant library at site G101 (L4). The mutant library 5 was a combination mutant library at amino acid sites G98 and Q99 (L5). The mutant library 6 was a combination mutant library at amino acid sites G100 and G101 (L6). The mutant library 7 was a combination mutant library at amino acid sites G98, Q99, G100 and G101 (L7).

A pair of primers was designed for the mutant library L1:

Forward Primer F1:
5'-GATAAATCTATTNNKCAGGGAGGCATAGGTGGCCCTATTG-3' (SEQ ID NO: 14)
Reverse Primer R1:
5'-TAGGGCCACCTATGCCTCCCTGMNNAATAGATTTATCTCC-3' (SEQ ID NO: 15)

A pair of primers was designed for the mutant library L2:

Forward Primer F2:
5'-GATAAATCTATTGGANNKGGAGGCATAGGTGGCCCTATTG-3' (SEQ ID NO: 16)
Reverse Primer R2:
5'-TAGGGCCACCTATGCCTCCMNNTCCAATAGATTTATCTCC-3' (SEQ ID NO: 17)

A pair of primers was designed for the mutant library L3:

Forward Primer F3:
5'-GATAAATCTATTGGACAGNNKGGCATAGGTGGCCCTATTG-3' (SEQ ID NO: 18)
Reverse Primer R3:
5'-TAGGGCCACCTATGCCMNNCTGTCCAATAGATTTATCTCC-3' (SEQ ID NO: 19)

A pair of primers was designed for the mutant library L4:

Forward Primer F4:
5'-GATAAATCTATTGGACAGGGANNKATAGGTGGCCCTATTG-3' (SEQ ID NO:20)
Reverse Primer R4:
5'-TAGGGCCACCTATMNNTCCCTGTCCAATAGATTTATCTCC-3' (SEQ ID NO: 21)

A pair of primers was designed for the mutant library L5:

Forward Primer F5:
5'-GATAAATCTATTNNKNNKGGAGGCATAGGTGGCCCTATTG-3' (SEQ ID NO: 22)
Reverse Primer R5:
5'-TAGGGCCACCTATGCCTCCMNNMNNAATAGATTTATCTCC-3' (SEQ ID NO: 23)

A pair of primers was designed for the mutant library L6:

Forward Primer F6:
5'- GATAAATCTATTGGACAGNNKNNKATAGGTGGCCCTATTG-3' (SEQ ID NO: 24)
Reverse Primer R6:
5'-TAGGGCCACCTATMNNMNNCTGTCCAATAGATTTATCTCC-3' (SEQ ID NO: 25)

A pair of primers was designed for the mutant library L7:

Forward Primer F7:
5'-GAAGGAGATAAATCTATTNNKNNKNNKNNKATAGGTGGCCCTATTGTTGATA-3' (SEQ ID NO:26)
Reverse Primer R7:
5'-CAATAGGGCCACCTATMNNMNNMNNMNNAATAGATTTATCTCCTTCATAAGT-3' (SEQ ID NO: 27)

In nucleic acid sequences, "N" is A, C, G, or T; "K" is G or T; and "M" is A or C.

Table 8 shows a reaction system for carrying out the error-prone PCR, and Table 9 shows reaction conditions, with 30 PCR cycles.

[Table 8]

| Components | Volume (μL) |
|---|---|
| 2x KOD FX Neo PCR buffer solution | 25 |
| 2 mM dNTPs | 10 |
| 10 μM Primer F | 1.5 |
| 10 μM Primer R | 1.5 |
| pCold WT-NS Pxluc template DNA (10 ng/μL) | 2.5 |
| KOD FX Neo enzyme | 1 |
| PCR grade water | 8.5 |
| Total Volume | 50 |

[Table 9]

| Phase | Temperature | Time | Number of cycles |
|---|---|---|---|
| Pre-denaturation | 94°C | 2min | 1 |
| Degeneration | 98°C | 10 s | 30 |
| Annealing | 58°C | 30 s | |
| Extension | 68°C | 5min | |
| Extension | 68°C | 5min | 1 |

After completion of the reaction, the template was digested by adding 0.5 μL of DpnI enzyme to the reaction system and incubating at 37°C for 3 h. The digested product was then transformed into DH5a competent cells and 10 ml of the plasmid was extracted overnight culture. After the transformation of the extracted plasmid into OrigamiB (DE3) chemically competent cells (WEIDI, EC1020S), the cells were plated and a single colony was picked from the plate and incubated overnight at 37°C. On the next day, the cells were diluted at a ratio of 1: 100 and transferred to 0.4 ml of fresh LB medium containing ampicillin resistance (100 μg/ml). The cells were incubated at 37°C with shaking at 200 rpm until OD600 was about 0.5 to 0.6, and cooled on ice for 1 h. The inducer IPTG was added at a final concentration of 1 mM and induced overnight at 16°C.

50 μL of the induced bacterial solution was added into a black 96-well plate, and then 10 μL of the substrates with a final concentration of 100 μM, i.e., CTZ (purchased from Baiaolaibo), fluorinated coelenterazine, ZS2, and ZS26, were added, respectively. The luminescence intensity was read from the luminescence module with a microplate reader to perform bacterial solution screening of mutants (screening criteria: the screening was performed based on a ratio of the luminescence intensity; when the ratio of the luminescence intensity of substrate ZS2 or ZS26 to the luminescence intensity of substrate F-CTZ is greater than the ratio of the luminescence intensity of wild type to the luminescence intensity of two substrates, it can be regarded as the dominant mutant).

**Example 4: Expression and Purification of Mutant Library of *Pleuromamma xiphia* luciferase**

1. Prokaryotic expression of *Pleuromamma xiphia* luciferase mutants

The mutant bacterial solutions screened from the mutant libraries, pCold-no sp Pxluc L1, pCold-no sp Pxluc L2, pCold-no sp Pxluc L3, pCold-no sp Pxluc L4, and pCold-no sp Pxluc L5, were cultured overnight at 37°C, diluted at a ratio of 1:100 on the next day, transferred into 15 ml fresh LB medium containing ampicillin (100 μg/ml), cultured at 37°C with shaking at 200 rpm until OD600 about 0.5 to 0.6, and cooled on ice for 1 h. The inducer IPTG was added at a final concentration of 1 mM and induced overnight at 16°C.

2. Purification of *Pleuromamma xiphia* luciferase mutants

The induced bacterial liquid precipitate was collected by centrifugation at a speed of 8,000 rpm/min for 10 min, and added with 600 μL of binding buffer solution (50 mM Tris, pH 8.0, 250 mM NaCl) and 6 μL of lysozyme. The mixture was lysed on ice for 30 min, disrupted by ultrasound (turning on for 2 seconds, turning off for 3 seconds, 60% power) for 10 min, and the supernatant (cell lysate) and precipitate were separated by centrifugation at 12,000 rpm for 30 min at 4°C.

50 μL of HisTrap FF filler was added to the manual column (purchased from Sangon Biotech, model F506607-0001# affinity chromatography column empty column), and the equilibrated filler was washed with 3 ml of binding buffer solution. 600 μL of filtered cell lysate was then added. After washing 10 times (3ml/time) with rinsing solution (50 mM Tris, pH 8.0, 250 mM NaCl, 10 mM imidazole), the protein was eluted with 100 μL of eluent (50 mM Tris, pH 8.0, 250 mM NaCl, 300 mM imidazole), and the eluted protein was collected.

The protein eluted from the Ni column was dialyzed using dialysis buffer solution (25 mM Tris, pH 8.0, 250 mM NaCl) at 4°C overnight. Protein concentration and purity profiles were determined by the BCA quantification kit (Thermo Scientific™ Pierce™ BCA Protein Assay Kit) and the SDS-PAGE method.

**Example 5: Substrate specificity assay of dominant mutants of *Pleuromamma xiphia* luciferase**

Protein concentration was accurately determined using the BCA quantification kit (Thermo Scientific™ Pierce™ BCA Protein Assay Kit), and luciferase was diluted to 1 μg/ml with diluent (50 mM Tris-HCl pH 8.0, 100 mM NaCl, 0.1% (v/v) Tween-20) and 10 μL was added to black 96-well plates, which was then added with 90 μL of the respective substrates, coelenterazine (purchased from Baiaolaibo), fluorinated coelenterazine, and coelenterazine derivatives ZS2 and ZS26

that were diluted to 100 μM with the same solution, and the luminescence intensity was read from the luminescence module with a microplate reader. The substrate specificity of the mutants was compared with the activity ratio of ZS2/F-CTZ and ZS26/F-CTZ. The activity assay results of the dominant specific mutants are shown in FIG. 7 and FIG. 8. Table 10 below lists results of the mutation sites and substrate specificity of ZS26/F-CTZ and ZS2/F-CTZ of the dominant mutant of *Pleuromamma xiphia* luciferase.

[Table 10]

| No. | Mutation site of mutant | Fold (ratio of RLU $\frac{ZS2}{F-CTZ}$ ) ZS2 luminescence intensity to F-CTZ luminescence intensity | Fold (ratio of RLU $\frac{ZS26}{F-CTZ}$ ) ZS26 luminescence intensity to F-CTZ luminescence intensity |
|---|---|---|---|
| pCold WT-NS Pxluc | Wild type | 3.56 | 1.77 |
| P25-1 | G98L, Q99R | 21.808 | 23.647 |
| P25-4 | G98P | 7.476 | 15.032 |
| P25-25 | G98Q | 5.932 | 2.492 |
| P25-30 | G98S, Q99W | 24.750 | 25.221 |
| P25-31 | Q991 | 10.543 | 6.047 |
| P25-32 | Q99Y | 3.841 | 74.857 |
| P25-46 | Q99A | 2.824 | 3.918 |
| P25-48 | Q99L | 2.994 | 9.601 |
| P25-49 | Q99F | 2.022 | 4.259 |
| P25-51 | G98L, Q99V | 23.173 | 46.976 |
| P25-62 | G98T, Q99P | 12.651 | 24.873 |
| P25-65 | G98L, Q99E | 14.841 | 43.355 |
| P26-1 | Q99M, G100S | 3.419 | 15.041 |
| P26-3 | G100Q, G101F | 6.21 | 7.71 |
| P26-4 | G100R, G101R | 5.83 | 8.22 |
| P26-5 | G100W, G101F | 8.16 | 6.36 |
| P26-7 | G100S, | 4.072 | 3.912 |
| P26-9 | G100T, G101S | 6.53 | 7.64 |
| P26-10 | G100R, G101C | 3.88 | 5.98 |
| P26-19 | G100A, G101R | 2.85 | 5.36 |

(continued)

| No. | Mutation site of mutant | Fold (ratio of RLU $\frac{ZS2}{F-CTZ}$) ZS2 luminescence intensity to F-CTZ luminescence intensity | Fold (ratio of RLU $\frac{ZS26}{F-CTZ}$) ZS26 luminescence intensity to F-CTZ luminescence intensity |
|---|---|---|---|
| P26-29 | G100L, G101Y | 5.20 | 7.83 |
| P26-34 | G100S, G101L | 2.21 | 3.97 |
| P26-55 | G101I | 3.76 | 4.06 |
| P26-62 | G100L, G101K | 3.56 | 6.17 |
| P26-64 | G100T | 3.57 | 5.97 |
| P26-65 | G100A, G101V | 2.89 | 4.79 |
| P26-95 | G100A, G101P | 1.97 | 4.00 |

The results in Table 10 and FIG. 7 and FIG. 8 reveal that: the wild type *Pleuromamma xiphia* luciferase without a signal peptide had a substrate specificity of about 1.77-fold for ZS26/F-CTZ and about 3.56-fold for ZS2/F-CTZ; the dominant mutant obtained by modifying the enzyme had an increased specificity for the substrate ZS26/F-CTZ, with the highest being about 74.857 times; its specificity for the substrate ZS2/F-CTZ was increased, with the highest being about 24.750 times.

**Example 6: Design and construction of *Pleuromamma xiphia* luciferase plasmid to be expressed in eukaryotic cells**

*Pleuromamma xiphia* luciferase containing a signal peptide used in the pEE12.4 vector was synthesized by the PCR technique, having the gene sequence as set forth in SEQ ID NO: 28. The signal peptide at N terminal has a histidine tag (6 x His) for purification, and the C terminal had an Avitag for biotinylating.

ATGTATATAAAAGTTTGGTTTGGTCTGGCTTGTCTTTCATTGGTTCTGGCC caccaccaccaccatcacggcagcCAACCAACTGAAAACAAGCAGGAGTCTCATATTGTAGATT CAGATCTTGATGGCGACCGTGGTAGGAAGTTGCCCGGAAAAAAGCTTCCTATAGA AGTACTCAAAATCATGGAAGCCAATGCCAGGAGAGCTGGTTGCACTAGAGGATGT CTCATATGTCTTTCAAAAATCAAGTGTACAGCCAAAATGAAGCGATACATTCCAGG GAGATGTCATACTTATGAAGGAGATAAATCTATTGGACAGGGAGGCATAGGTGGCC CTATTGTTGATATTCCTGAAATTATTGGATTCAAGAACATGGAACCCATGGATCAGT TCATCGCACAAGTTGATCTGTGCGCCGACTGTACAACTGGGTGCCTGAAAGGCCTT GCTAATGTTAGGTGCAATGACTTGCTGAAGAAATGGCTGCCTGACAGATGTGCTGG TTTTGCCGACAAAATTCAAAATGAAGTGGATAGTATCAAGGGcatggctggggatcgcggctc cggactgaatgatatc (SEQ ID NO: 28)

Plasmid for eukaryotic expression of *Pleuromamma xiphia* luciferase adopted prokaryotic expression plasmid pET28a Pxluc WT containing a signal peptide as the template DNA. The sequences of the primers used in the PCR reaction for constructing the inserted fragment of the expression plasmid are shown in Table 11. The reaction system is shown in Table 12. The reaction conditions are shown in Table 13.

[Table 11]

| Name | Sequence |
|---|---|
| Forward Primer insert-F | 5'-CACCACCATCACGGCAGCCAACCAACTGAAAACAAG-3' (SEQ ID NO: 29) |
| Reverse Primer insert-R | 5'-GATATCATTCAGTCCGGAGCCGCGATCCCCAGCCATG-3' (SEQ ID NO: 30) |

[Table 12]

| Components | Volume (μL) |
|---|---|
| 2x KOD FX Neo PCR buffer solution | 25 |
| 2 mM dNTPs | 10 |
| 10 μM Primer insert-F | 1.5 |
| 10 μM Primer insert-R | 1.5 |
| Template DNA pET28a Pxluc WT (10 ng/μL) | 2.5 |
| KOD FX Neo enzyme | 1 |
| PCR grade water | 8.5 |
| Total Volume | *50* |

[Table 13]

| Phase | Temperature | Time | Number of cycles |
|---|---|---|---|
| Pre-denaturation | 94°C | 2 min | 1 |
| Degeneration | 98°C | 10 s | 30 |
| Annealing | 58°C | 30 s | |
| Extension | 68°C | 5 min | |
| Extension | 68°C | 5 min | 1 |

The template was digested by adding 0.5 μL of DpnI enzyme to the reaction and incubating for 3 h at 37°C, and then the product of about 528 bp was recovered as inserted fragment, i.e., insert.

The vector was linearized by means of PCR using the pEE12.4 vector as a template to facilitate recombination with the inserted fragment. By using KOD FX neo enzymes, PCR reaction system was prepared and PCR reaction was performed according to the manual thereof. The sequences of primers used are shown in Table 14. The PCR reaction system is shown in Table 15. The PCR reaction conditions are shown in Table 16.

[Table 14]

| Name | Sequence (5 -3) |
|---|---|
| Forward primer | 5'-CATGGCTGGGGATCGCGGCTCCGGACTGAATGATATC-3' (SEQ ID NO: 31) |
| Reverse primer | 5'-CTTGTTTTCAGTTGGTTGGCTGCCGTGATGGTGGTG-3' (SEQ ID NO: 32) |

[Table 15]

| Components | Volume (μL) |
|---|---|
| 2x KOD FX Neo PCR buffer solution | 25 |
| 2 mM dNTPs | 10 |

(continued)

| Components | Volume (μL) |
|---|---|
| 10 μM Primer vector-F | 1.5 |
| 10 μM Primer vector-R | 1.5 |
| pEE12.4 plasmid (10 ng/μL) | 2.5 |
| KOD FX Neo | 1 |
| PCR grade water | 8.5 |
| Total Volume | *50* |

[Table 16]

| Phase | Temperature | Time | Number of cycles |
|---|---|---|---|
| Pre-denaturation | 94°C | 2 min | 1 |
| Degeneration | 98°C | 10 s | 30 |
| Annealing | 58°C | 30 s | |
| Extension | 68°C | 5min | |
| Extension | 68°C | 5min | 1 |

The template was digested by adding 0.5 μL of DpnI enzyme to the reaction system and incubating at 37°C for 3 h, and then the product of about 7712 bp was recovered as a linearized vector.

The insert obtained in the present example was recombined with the vector using the Takara In-Fusion Cloning kit according to the reaction system shown in Table 17, and the reaction condition was 50°C for 15 min.

[Table 17]

| Components | Volume (μL) |
|---|---|
| insert (10 ng/μL) | 5 |
| vector (10 ng/μL) | 3 |
| Premix | 2 |
| Total Volume | 10 |

2.5 μL of the above reaction product was transformed into DH5α competent cells and spread onto plates containing ampicillin resistance at a final concentration of 100 μg/mL. On the next day, a single clone was picked from the plates, and plasmids were extracted. Sequencing was performed to ensure that the target fragment was correctly inserted into the vector and that the resulting plasmid was the wild-type luciferase pEE12.4-Pxluc WT for eukaryotic expression. The resulting plasmid was tested and the detected plasmid profile is shown in FIG. 9, which is consistent with the expectation.

The mutant plasmid pEE12.4 Pxluc P26-95 for eukaryotic expression was synthesized by PCR method using the constructed pEE12.4-Pxluc WT as template DNA. The sequences of primers are shown in Table 18. The PCR reaction system is shown in Table 19. The PCR reaction conditions are the same as those in Table 16.

[Table 18]

| Name | Sequence (5 -3) |
|---|---|
| Forward primer | 5'-GATAAATCTATTGGACAGGCGCCTATAGGTGGCCCTATTG-3' (SEQ ID NO: 33) |
| Reverse primer | 5'-TAGGGCCACCTATAGGCGCCTGTCCAATAGATTTATCTCC -3' (SEQ ID NO: 34) |

[Table 19]

| Components | Volume (μL) |
|---|---|
| 2x KOD FX Neo PCR buffer solution | 25 |
| 2 mM dNTPs | 10 |
| 10 μM Primer vector-F | 1.5 |
| 10 μM Primer vector-R | 1.5 |
| pEE12.4 Pxluc P26-95 (10 ng/μL) | 2.5 |
| KOD FX Neo | 1 |
| PCR grade water | 8.5 |
| Total Volume | *50* |

After completion of the reaction, the template was digested by adding 0.5 μL of DpnI enzyme to the reaction system, and incubating at 37°C for 3 h. 2.5 μL of the digested reaction product was transformed into DH5α competent cells and plated on ampicillin-resistant plates containing a final concentration of 100 μg/mL. On the next day, a single clone was picked from the plates, and plasmids were extracted. Sequencing was performed to ensure that the mutant sequence was correct and the resulting plasmid was the mutated luciferase pEE12.4 Pxluc P26-95 (luciferase mutant expression vector containing a signal peptide sequence, mutation sites G100A and G101P) for eukaryotic expression.

**Example 7: Eukaryotic expression and protein purification of *Pleuromamma xiphia* luciferase**

According to the instructions of ExpiFectamine™ CHO transfection kit (Gibco, A29129), pEE12.4 Pxluc WT and mutant pEE12.4 Pxluc P26-95 plasmids obtained in Example 6 were transfected into 30 mL of Expi-CHO cells, respectively. Seven days after transfection, cell viability was measured less than 90%, and supernatants were collected after centrifugation at 8,000 rpm for 10 min at 4°C.

2 mL of HisTrap FF filler was added to the manual column (purchased from Sangon Biotech, model F506607-0001# affinity chromatography column empty column) respectively and the equilibrated filler was washed with 30 mL of binding buffer solution. Approximately 30 mL of filtered cell supernatant was then added separately. After washing 10 times (10 mL/time) with washing solution (50 mM Tris, pH 8.0, 250 mM NaCl, 10 mM imidazole) and then eluting 4 to 5 times with 500 μL of eluent (50 mM Tris, pH 8.0, 250 mM NaCl, 300 mM imidazole), the eluted proteins were collected separately. The eluted protein was detected by 12% SDS-PAGE, to obtain relatively pure Pxluc protein. The results of purification are shown in FIG. 10.

**Example 8: Coupling and activity assay of dominant mutants of *Pleuromamma xiphia* luciferase**

The Pxluc proteins obtained in Example 7, containing the AviTag tag, i.e., AviTag-Pxluc-WT and mutant AviTag-Pxluc-P26-95, were biotinylated as Biotin-Avitag-Pxluc-WT and Biotin-AviTag-Pxluc-P26-95 by BirA enzyme (Avidity, BIRA500) in the reaction system shown in Table 18.

[Table 20]

| Components | Volume (μL) |
|---|---|
| AviTag-Pxluc (1 mg/mL) | 12 |
| BiomixA | 10 |
| BiomixB | 10 |
| BirA (1 mg/mL) | 3 |
| $H_2O$ | 65 |
| Total Volume | 100 |

SA-Pxluc can be prepared by adding SA to the system shown in Table 20, and the histidine tag on Pxluc can be used for further purification to obtain relatively pure SA-Pxluc. The purification results are shown in FIG. 11.

pEE12.4 Pxluc WT and mutant pEE12.4 Pxluc P26-95, as well as the coupled products SA-Pxluc WT and SA-Pxluc P26-95 were subjected to the activity assay according to the method described in Example 5. The results are shown in FIG. 12. FIG. 12 indicates that: both Pxluc WT and mutant Pxluc P26-95 that were expressed in eukaryotic cells had the activity of catalyzing the substrates of coelenterazine derivatives ZS2, ZS26; and compared with Pxluc WT, the mutant Pxluc P26-95 had significantly improved substrate specificity to ZS26/F-CTZ and improved substrate specificity to ZS26/F-CTZ. The substrate specificity of the coupled product SA-Pxluc P26-95 to ZS26/F-CTZ was significantly improved compared with SA-Pxluc WT.

In the specification, references to descriptions of the term "an embodiment", "some embodiments", "examples", "specific examples", or "some examples", etc. means that a particular feature, structure, material, or characteristic described in connection with the embodiment or example is included in at least an embodiment or example of the present disclosure. In this specification, schematic representations of the above terms are not necessarily directed to the same embodiment or example. Furthermore, the particular features, structures, materials, or characteristics described may be combined in any suitable manner in one or more embodiments or examples. Furthermore, combinations and combinations of the various embodiments or examples and features of the various embodiments or examples described in this specification can be made by those skilled in the art without departing from the scope of the present disclosure.

While embodiments of the present disclosure have been illustrated and described above, it can be understood that the above-mentioned embodiments are illustrative and not restrictive and that those skilled in the art can make changes, modifications, substitutions, and variations to the above embodiments without departing from the scope of the present disclosure.

## Claims

**1.** A mutated luciferase, having at least one of the following mutation sites based on the amino acid sequence as set forth in SEQ ID NO: 2: sites 98, 99, 100, and 101, and the mutated luciferase comprising or not comprising a signal peptide amino acid sequence.

**2.** The mutated luciferase according to claim 1, having one or more of the following mutations (1) to (4) based on the amino acid sequence as set forth in SEQ ID NO: 2:

(1) G at site 98 is mutated to L, P, Q, S, or T;
(2) Q at site 99 is mutated to R, W, I, Y, A, L, F, V, P, E, or M;
(3) G at site 100 is mutated to S, Q, R, W, T, A, or L; and
(4) G at site 101 is mutated to F, R, S, C, Y, L, I, K, V, or P.

**3.** The mutated luciferase according to claim 1, having one or two of the following mutation sites based on the amino acid sequence as set forth in SEQ ID NO: 2: sites 98, 99, 100 and 101, and the mutated luciferase comprising a signal peptide amino acid sequence or comprising no signal peptide amino acid sequence.

**4.** The mutated luciferase according to claim 3, having one or two of the following mutations (1) to (4) based on the amino acid sequence as set forth in SEQ ID NO: 2:

(1) G at site 98 is mutated to L, P, Q, S, or T;
(2) Q at site 99 is mutated to R, W, I, Y, A, L, F, V, P, E, or M;
(3) G at site 100 is mutated to S, Q, R, W, T, A, or L; and
(4) G at site 101 is mutated to F, R, S, C, Y, L, I, K, V, or P.

**5.** The mutated luciferase according to any one of claims 1 to 4, having the following mutations based on the amino acid sequence as set forth in SEQ ID NO: 2:

1) G at site 98 is mutated to L, and Q at site 99 is mutated to R; or
2) G at site 98 is mutated to P; or
3) G at site 98 is mutated to Q; or
4) G at site 98 is mutated to S, and Q at site 99 is mutated to W; or
5) Q at site 99 is mutated to I; or
6) Q at site 99 is mutated to Y; or
7) Q at site 99 is mutated to A; or
8) Q at site 99 is mutated to L; or
9) Q at site 99 is mutated to F; or

10) G at site 98 is mutated to L, and Q at site 99 is mutated to V; or
11) G at site 98 is mutated to T, and Q at site 99 is mutated to P; or
12) G at site 98 is mutated to L, and Q at site 99 is mutated to E; or
13) Q at site 99 is mutated to M, and G at site 100 is mutated to S; or
14) G at site 100 is mutated to Q, and G at site 101 is mutated to F; or
15) G at site 100 is mutated to R, and G at site 101 is mutated to R; or
16) G at site 100 is mutated to W, and G at site 101 is mutated to F; or
17) G at site 100 is mutated to S; or
18) G at site 100 is mutated to T, and G at site 101 is mutated to S; or
19) G at site 100 is mutated to R, and G at site 101 is mutated to C; or
20) G at site 100 is mutated to A, and G at site 101 is mutated to R; or
21) G at site 100 is mutated to L, and G at site 101 is mutated to Y; or
22) G at site 100 is mutated to S, and G at site 101 is mutated to L; or
23) G mutation at site 101 is I; or
24) G at site 100 is mutated to L, and G at site 101 is mutated to K; or
25) G at site 100 is mutated to T; or
26) G at site 100 is mutated to A, and G at site 101 is mutated to V; or
27) G at site 100 is mutated to A, and G at site 101 is mutated to P.

**6.** The mutated luciferase according to any one of claims 1 to 5, not comprising the signal peptide amino acid sequence.

**7.** A nucleic acid molecule, encoding the mutated luciferase according to any one of claims 1 to 6.

**8.** An expression vector, comprising the nucleic acid molecule according to claim 7.

**9.** A recombinant cell, carrying the nucleic acid molecule according to claim 7 or the expression vector according to claim 8.

**10.** The recombinant cell according to claim 9, wherein the recombinant cell is selected from the group consisting of *Escherichia coli,* yeast, and mammalian cells.

**11.** A method for producing a mutated luciferase, comprising:

introducing the expression vector according to claim 8 into recombinant cells;
culturing and propagating the recombinant cells; and
collecting product of said culturing and propagating, and extracting or purifying the mutated luciferase.

**12.** A method for detecting a nucleic acid sequence using the mutated luciferase according to any one of claims 1 to 6, the method comprising:

A) forming, by way of chemical coupling or bioconjugation or by means of fusion protein, a first mutated luciferase complex of a first specific recognition protein and the mutated luciferase according to any one of claims 1 to 6, and forming, using a second luciferase as a signal peptide, a second mutated luciferase complex of a second specific recognition protein and the second luciferase by way of chemical coupling or bioconjugation or by means of fusion protein;
B) reacting the first mutated luciferase complex with a first substrate to generate a first luminescent signal, and reacting the second luciferase complex with a second substrate to generate a second luminescent signal, wherein the first mutated luciferase complex and the second substrate do not have significant cross-substrate reaction, and wherein the second luciferase complex and the first substrate do not have significant cross-substrate reaction; and
C) determining four bases A, T, G, and C for target nucleic acid sequencing by detecting fluorescence signal and signal combination of a self-luminescence system of the mutated luciferase and the second luciferase.

**13.** The method according to claim 12, wherein:

when the mutated luciferase and the second luciferase are the same, the first substrate and the second substrate are the same; and
when the mutated luciferase and the second luciferase are different, the first substrate and the second substrate

are different.

**14.** A nucleic acid sequencing kit, comprising the mutated luciferase according to any one of claims 1 to 6.

**15.** A method for detecting a content of an analyte, comprising:

a) forming, using the mutated luciferase according to any one of claims 1 to 6 as a signal peptide, a complex of a specific recognition protein of the analyte and the mutated luciferase by way of chemical coupling or bioconjugation or by means of fusion protein;
b) contacting the analyte with the complex;
c) adding a substrate for *Pleuromamma xiphia* luciferase or an analogue of the substrate to the reaction system; and
d) determining the content of the analyte based on a fluorescence intensity of the reaction system detected subsequent to said adding the substrate for the *Pleuromamma xiphia* luciferase or the analogue of the substrate.

**16.** The method according to claim 15, wherein the substrate for the *Pleuromamma xiphia* luciferase is selected from at least one of coelenterazine, fluorinated coelenterazine, or coelenterazine derivative;
optionally, the coelenterazine derivative is selected from coelenterazine derivative ZS2 or coelenterazine derivative ZS26,

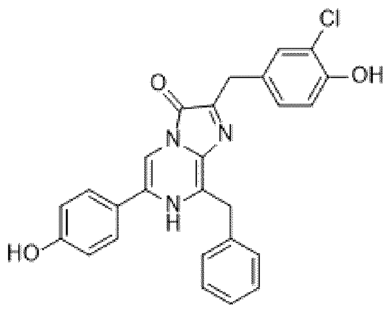

ZS2

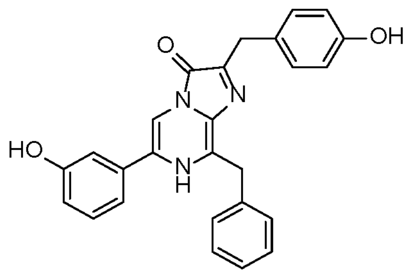

ZS26.

**17.** A method for screening a substrate for *Pleuromamma xiphia* luciferase, comprising:

I) contacting the mutated luciferase according to any one of claims 1 to 6 with a substrate to be screened to obtain a reaction mixture; and
II) determining, based on whether a chemical light signal is emitted by the reaction mixture obtained in step I), whether the substrate to be screened is a target substrate.

**18.** The method according to claim 17, wherein an indicator that the substrate to be screened is the target substrate is that a chemical light signal emitted by the reaction mixture obtained in step I).

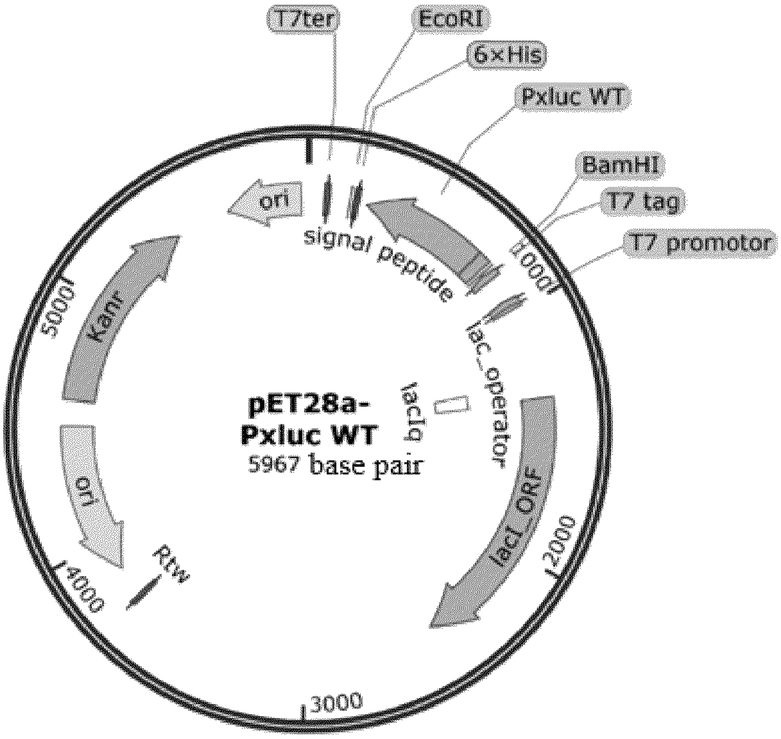
T7ter
EcoRI
6×His
Pxluc WT
BamHI
T7 tag
T7 promotor
ori
signal peptide
1000
lac_operator
lacIq
lacI_ORF
2000
3000
4000
Rtw
ori
5000
Kanr
pET28a-
Pxluc WT
5967 base pair

FIG. 1

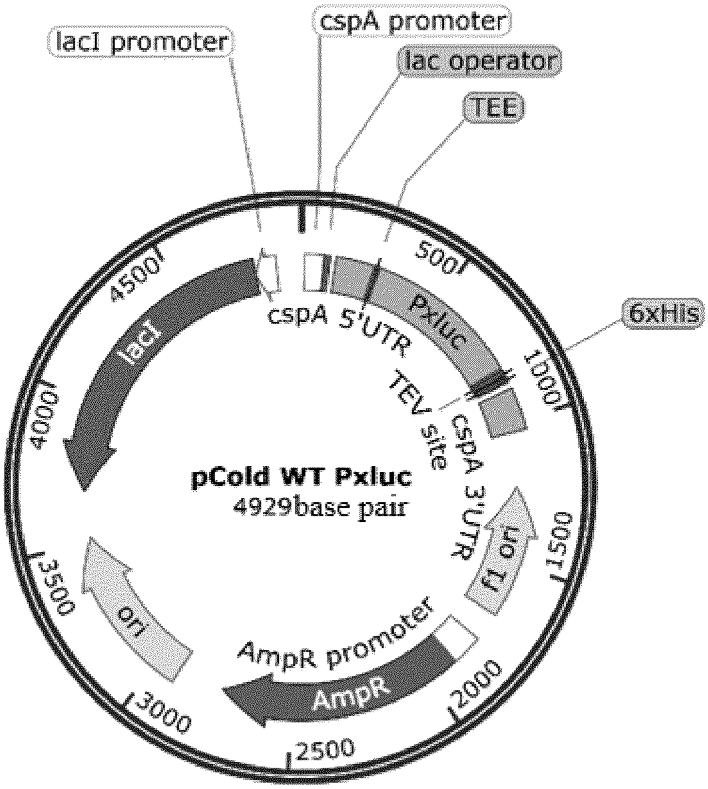
lacI promoter
cspA promoter
lac operator
TEE
6xHis
500
Pxluc
cspA
5'UTR
TEV site
1000
cspA 3'UTR
f1 ori
1500
AmpR promoter
AmpR
2000
2500
3000
ori
3500
4000
lacI
4500
pCold WT Pxluc
4929base pair

FIG. 2

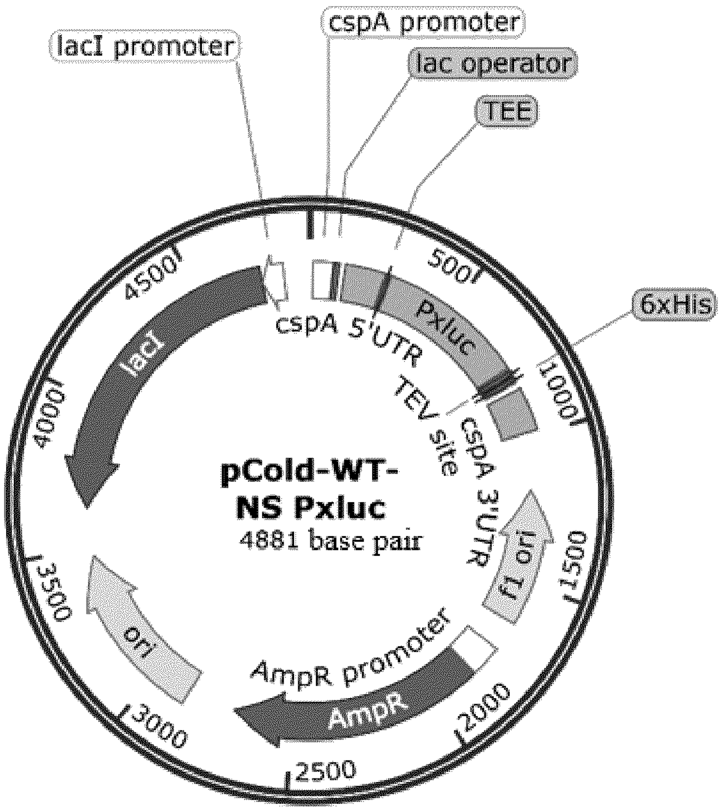
lacI promoter
cspA promoter
lac operator
TEE
6xHis
500
1000
1500
2000
2500
3000
3500
4000
4500
cspA 5'UTR
Pxluc
TEV site
cspA 3'UTR
f1 ori
AmpR promoter
AmpR
ori
lacI
pCold-WT-NS Pxluc
4881 base pair

FIG. 3

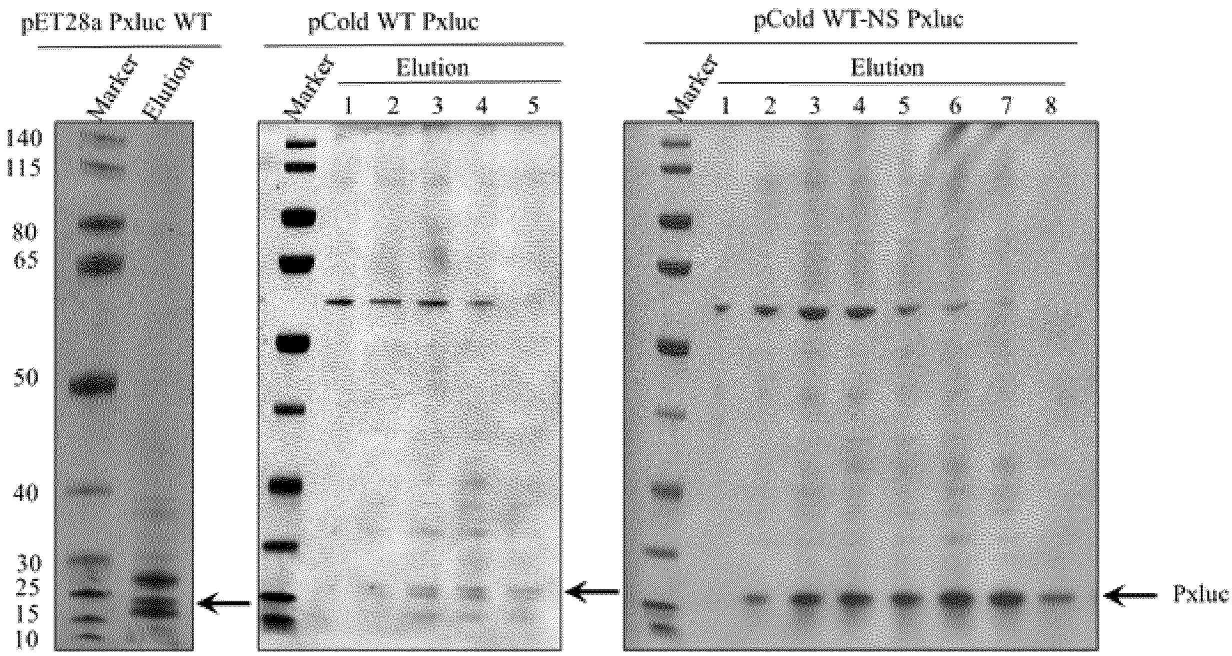
pET28a Pxluc WT
Marker
Elution
pCold WT Pxluc
Marker
Elution
1 2 3 4 5
pCold WT-NS Pxluc
Marker
Elution
1 2 3 4 5 6 7 8
140
115
80
65
50
40
30
25
15
10
Pxluc

FIG. 4

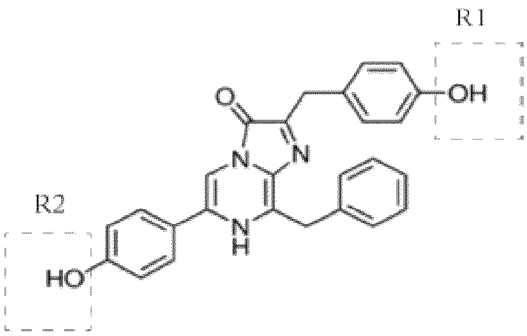

A

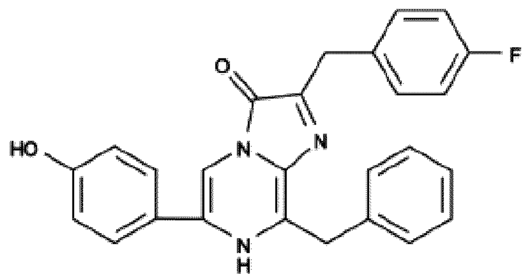

B

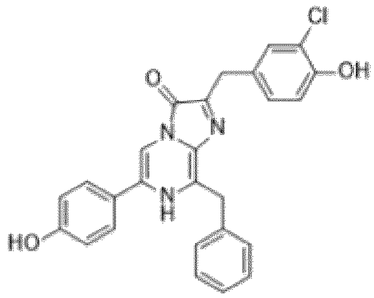

C

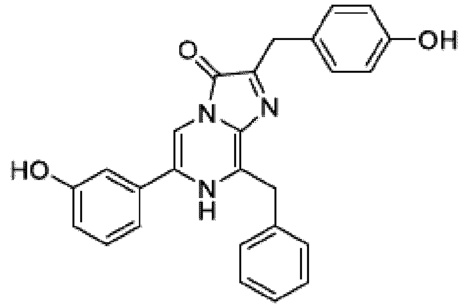

D

FIG. 5

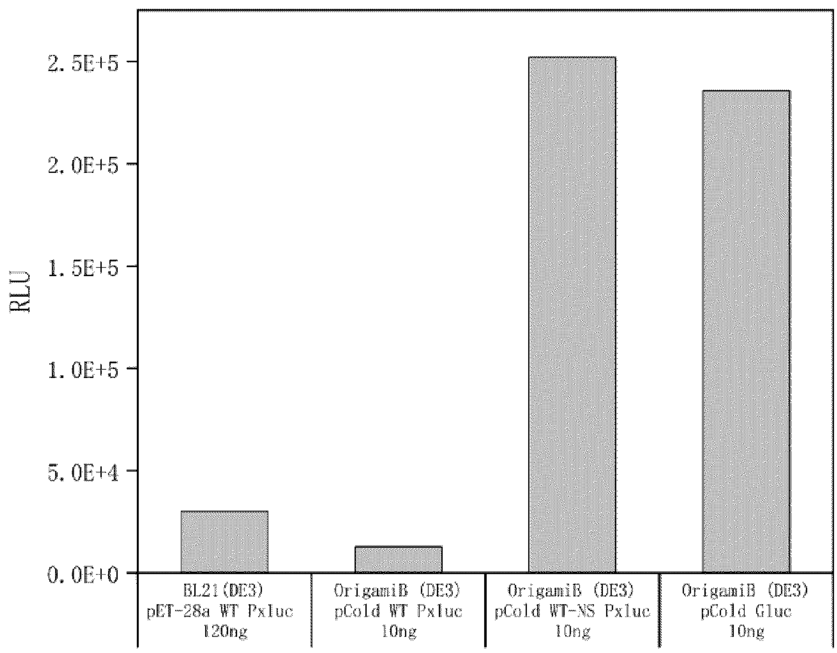


FIG. 6

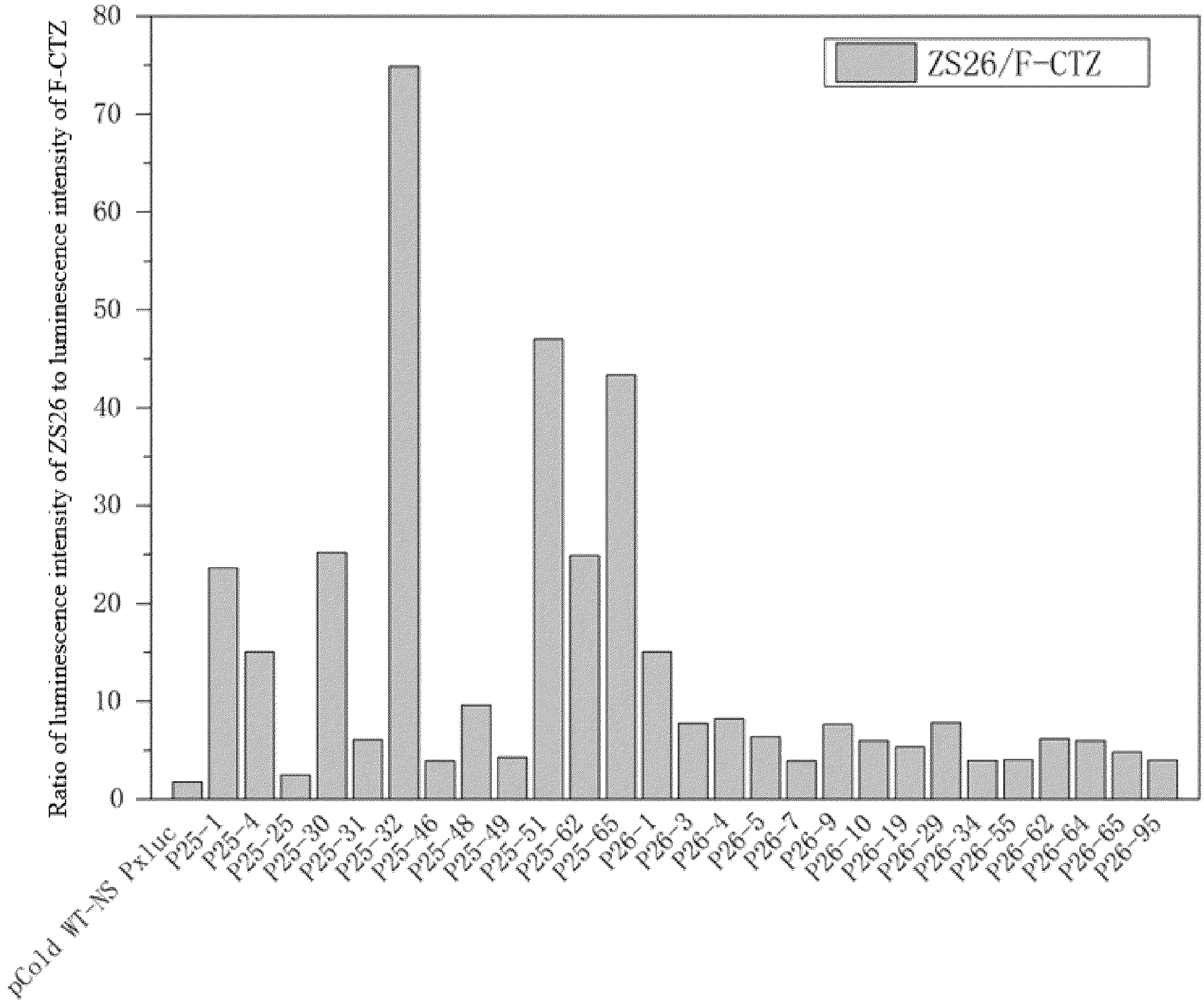
Ratio of luminescence intensity of ZS26 to luminescence intensity of F-CTZ
80
70
60
50
40
30
20
10
0
ZS26/F-CTZ
pCold WT-NS Pxluc
P25-1
P25-4
P25-25
P25-30
P25-31
P25-32
P25-46
P25-48
P25-49
P25-51
P25-62
P25-65
P26-1
P26-3
P26-4
P26-5
P26-7
P26-9
P26-10
P26-19
P26-29
P26-34
P26-55
P26-62
P26-64
P26-65
P26-95

FIG. 7

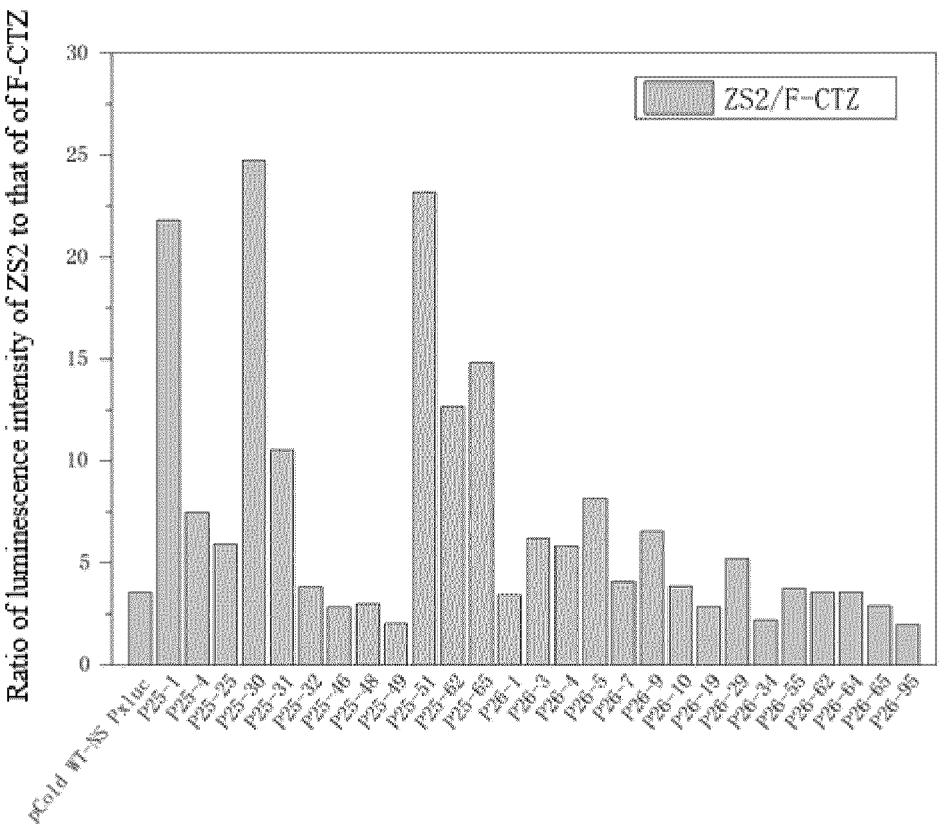

Ratio of luminescence intensity of ZS2 to that of F-CTZ
ZS2/F-CTZ
30
25
20
15
10
5
0
pCold WT-NS Pxluc
P25-1
P25-4
P25-25
P25-30
P25-31
P25-32
P25-46
P25-48
P25-49
P25-51
P25-62
P25-65
P26-1
P26-3
P26-4
P26-5
P26-7
P26-9
P26-10
P26-19
P26-29
P26-34
P26-55
P26-62
P26-64
P26-65
P26-96


FIG. 8

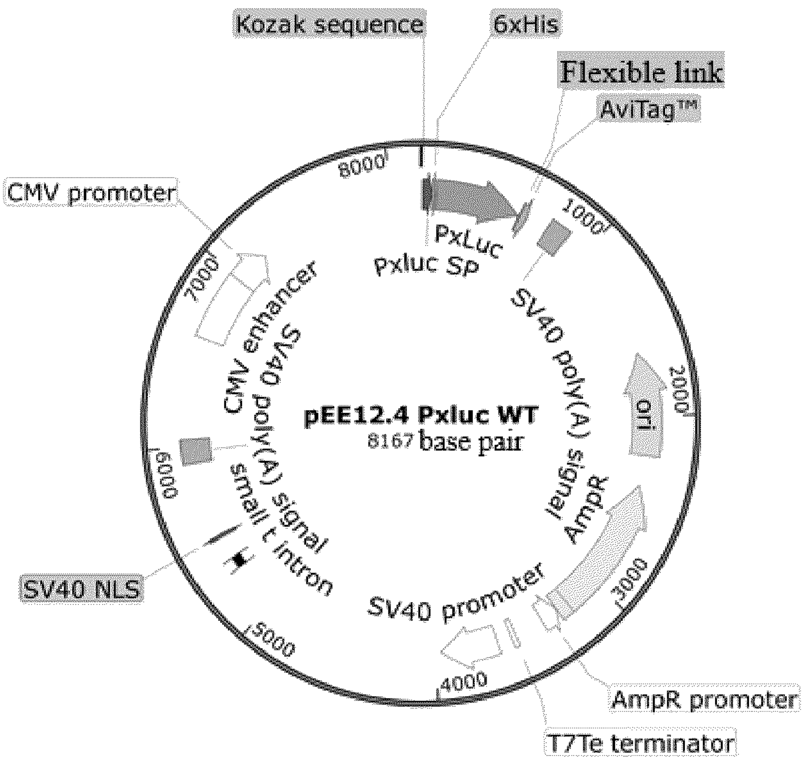

Kozak sequence
6xHis
Flexible link
AviTag™
CMV promoter
8000
1000
PxLuc
Pxluc SP
7000
CMV enhancer
SV40 poly(A) signal
2000
ori
pEE12.4 Pxluc WT
8167 base pair
SV40 poly(A) signal
AmpR
6000
small t intron
SV40 NLS
3000
SV40 promoter
5000
4000
AmpR promoter
T7Te terminator


FIG. 9

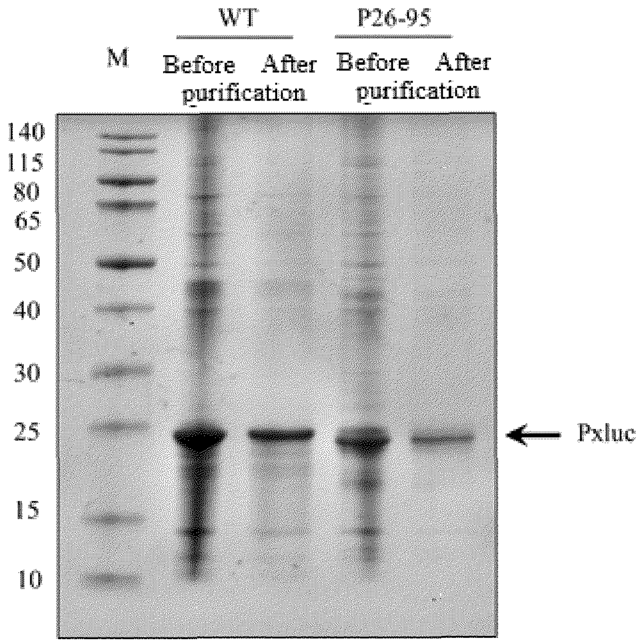
WT
P26-95
M
Before After
purification
Before After
purification
140
115
80
65
50
40
30
25
15
10
Pxluc

FIG. 10

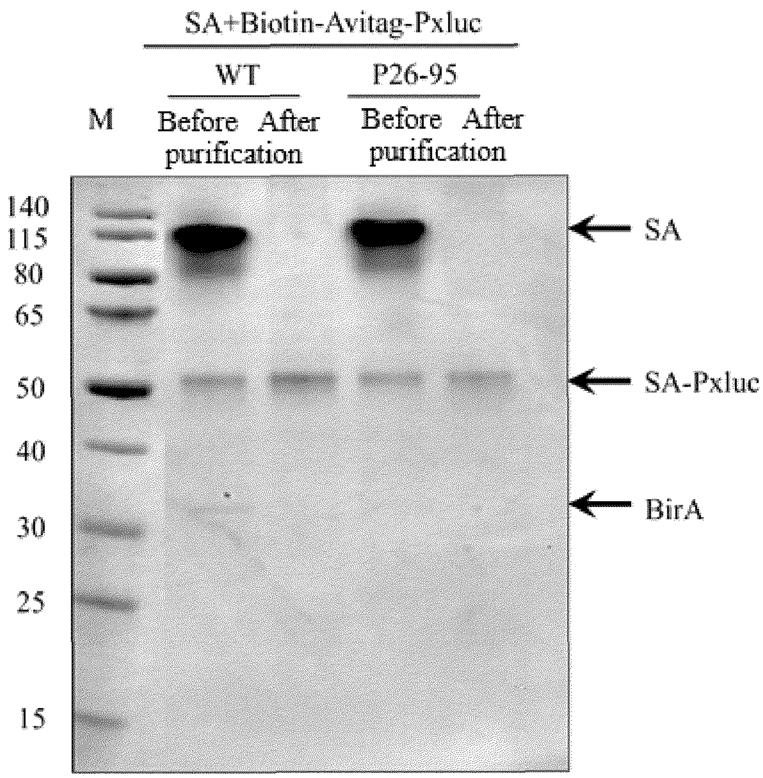
SA+Biotin-Avitag-Pxluc
WT
P26-95
M
Before After
purification
Before After
purification
140
115
80
65
50
40
30
25
15
SA
SA-Pxluc
BirA

FIG. 11

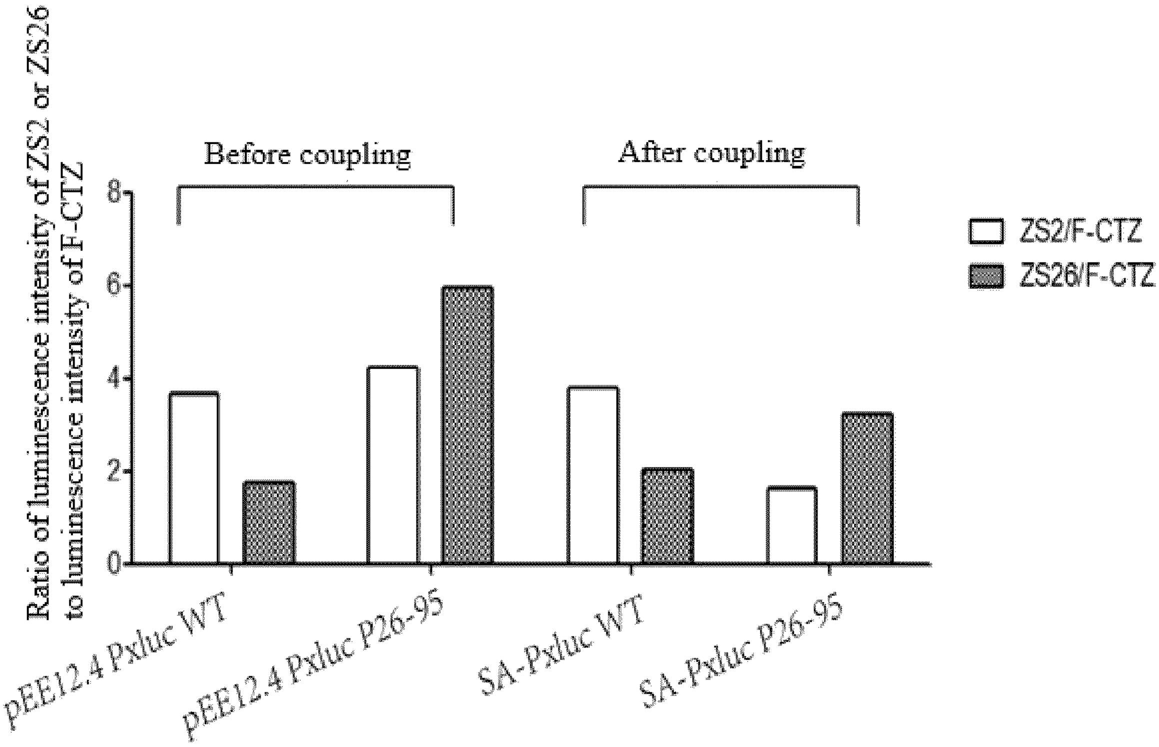
Before coupling
After coupling
Ratio of luminescence intensity of ZS2 or ZS26 to luminescence intensity of F-CTZ
8
6
4
2
0
ZS2/F-CTZ
ZS26/F-CTZ
pEE12.4 Pxluc WT
pEE12.4 Pxluc P26-95
SA-Pxluc WT
SA-Pxluc P26-95

FIG. 12

**INTERNATIONAL SEARCH REPORT**

International application No. **PCT/CN2022/102827**

**A. CLASSIFICATION OF SUBJECT MATTER**

C12N 15/53(2006.01)i；C12N 9/02(2006.01)i；C12N 15/62(2006.01)i；C07K 14/02(2006.01)i

According to International Patent Classification (IPC) or to both national classification and IPC

**B. FIELDS SEARCHED**

Minimum documentation searched (classification system followed by classification symbols)

C12N; C07K

Documentation searched other than minimum documentation to the extent that such documents are included in the fields searched

Electronic data base consulted during the international search (name of data base and, where practicable, search terms used)

CNTXT, ENTXT, VEN, CJFD, CNKI, PUBMED, ISI-WEB OF SCIENCE, 百度学术, BAIDU SCHOLAR, GenBank+DDBJ+EMBL, 中国专利生物序列检索数据库, Chinese Patent Biological Sequence Retrieval Database: 桡足类荧光素酶, 桡脚类荧光素酶, 剑乳点水蚤荧光素酶, 桡足类, 桡脚类, 剑乳点水蚤, 荧光素酶, 突变体, 突变, 第98位, 第99位, 第100位, 第101位, Pleuromamma xiphia, Gaussia princeps, Gaussia luciferase, luciferase, variant, mutant, mutate, mutation, G98, Q99, G100, G101, "98", "99", "100", "101", 对SEQ ID NO: 2所示序列的第98, 99, 100, 101位的突变进行序列检索, Sequence search for mutations at positions 98, 99, 100, and 101 of the sequence shown in SEQ ID NO: 2

**C. DOCUMENTS CONSIDERED TO BE RELEVANT**

| Category* | Citation of document, with indication, where appropriate, of the relevant passages | Relevant to claim No. |
|---|---|---|
| A | CN 105779472 A (CHONGQING MEDICAL UNIVERSITY) 20 July 2016 (2016-07-20)<br>see abstract, and claims 1-8 | 1-18 |
| A | CN 104781401 A (THE NATIONAL INSTITUTE OF ADVANCED INDUSTRIAL SCIENCE AND TECHNOLOGY) 15 July 2015 (2015-07-15)<br>see abstract | 1-18 |
| A | WO 2018169317 A1 (INDUSTRY-UNIVERSITY COOPERATION FOUNDATION HANYANG UNIVERSITY) 20 September 2018 (2018-09-20)<br>see abstract | 1-18 |
| A | US 2012122182 A1 (TANNOUS, B. A. et al.) 17 May 2012 (2012-05-17)<br>see abstract | 1-18 |

☑ Further documents are listed in the continuation of Box C. ☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **30 November 2022** | **11 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/102827**

**C. DOCUMENTS CONSIDERED TO BE RELEVANT**

| Category* | Citation of document, with indication, where appropriate, of the relevant passages | Relevant to claim No. |
|---|---|---|
| A | Thorsten Wille et al. "Gaussia princeps Luciferase as a Reporter for Transcriptional Activity, Protein Secretion, and Protein-Protein Interactions in Salmonella enterica Serovar Typhimurium" *Applied and Environmental Microbiology,* Vol. 78, No. 1, 21 October 2011 (2011-10-21), ISSN: 0099-2240, see page 250, and abstract | 1-18 |
| A | Takenaka Y. et al. "secreted luciferase [Pleuromamma xiphias]" *GenBank,* 02 October 2013 (2013-10-02), see sequence and information of accession number: BAN91832.1 | 1-18 |

Form PCT/ISA/210 (second sheet) (January 2015)

**INTERNATIONAL SEARCH REPORT**

International application No.
**PCT/CN2022/102827**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2022/102827**

| Patent document cited in search report | Publication date (day/month/year) | Patent family member(s) | Publication date (day/month/year) |
|---|---|---|---|
| CN 105779472 A | 20 July 2016 | None | |
| CN 104781401 A | 15 July 2015 | WO 2014065047 A1 | 01 May 2014 |
| | | EP 2913400 A1 | 02 September 2015 |
| | | US 2015284813 A1 | 08 October 2015 |
| WO 2018169317 A1 | 20 September 2018 | KR 20180105772 A | 01 October 2018 |
| US 2012122182 A1 | 17 May 2012 | WO 2011002924 A2 | 06 January 2011 |

Form PCT/ISA/210 (patent family annex) (January 2015)